# EUROPEAN PATENT APPLICATION

(11) **EP 2 210 890 A1**
(43) Date of publication of application: **28.07.2010**
(21) Application number: 09382004.1
(22) Date of filing: 19.01.2009
(51) Int. Cl.: C07D 413/04, C07D 413/14, A61K 31/435, A61P 37/06

(54) **Oxadiazole derivatives as S1P1 receptor agonists**

(71) Applicant: Almirall, S.A., 08022 Barcelona (ES)
(72) Inventor: Aguilar Izquierdo, Nuria, 08005 Barcelona (ES); CArrascal Riera, Marta, 08028 Barcelona (ES); Castro Palomino Laria, Julio Cesar, 08330 Premia de Mar (Barcelona) (ES); Erra Sola, Montserrat, 08903 L'Hospitalet de Llobregat (Barcelona) (ES)
(74) Representative: Elzaburu Marquez, Alberto

(57) **Abstract**

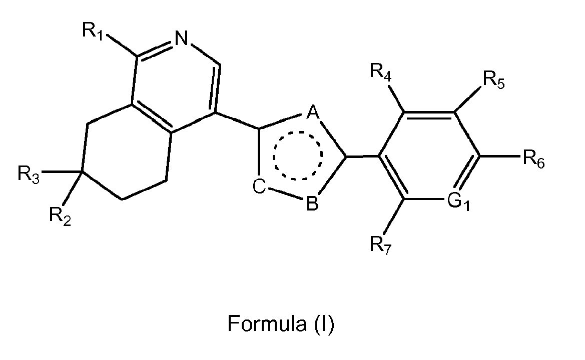

New compounds having the chemical structure of formula (I) or pharmaceutically acceptable salts or N-oxides thereof wherein
A is selected from the group consisting of -N-, -O- and -S-;
B and C independently are selected from the group consisting of -N- and -O-, with the proviso that at least two of A, B and C are nitrogen atoms;
G¹ is selected from the group consisting of nitrogen atoms and -CR^{C}- groups, wherein R^{C} represents a hydrogen atom, a halogen atom, a C₁₋₄ alkyl group or a C₁₋₄ alkoxy group;
R¹ is selected from the group consisting of hydrogen atoms, C₁₋₄ alkyl groups, C₁₋₄ alkoxy groups, C₃₋₄ cycloalkyl groups, and -NR^{d}R^{e} groups wherein R^{d} and R^{e} are independently selected from hydrogen atoms and C₁₋₄ alkyl groups;
R² and R³ are independently selected from the group consisting of hydrogen atoms and C₁₋₄ alkyl groups;
R⁴, R⁵ and R⁷ are independently selected from the group consisting of hydrogen atoms, halogen atoms, C₁₋₄ alkyl groups, C₁₋₄ alkoxy groups and C₁₋₄ haloalkyl groups;
R⁶ represents a C₁₋₄ alkyl group or a C₁₋₄ hydroxyalkyl group; or R⁶ is selected from the group consisting of -S(O)₂-NR^{a}R^{b} groups, -(CR^{f}R^{g})ₙ-(CR^{h}Rⁱ)ₓ-(CR^{j}R^{k})_{y}-NR^{a}R^{b} groups,
-(CH₂)ₙ-NR^{a}R^{b} groups, -O-(CH₂)ₙ-NR^{a}R^{b} groups, -(CH₂)ₙ-COOH groups, -(CH₂)ₙ-NR^{a}-CO-R^{b'} groups, -(CH₂)ₙ-NR^{a}-(CH₂)ₚ-(NH)_{q}-SO-CH₃ groups and -(CH₂)ₙ-CO-NR^{a}R^{b} groups, wherein
n, p, x and y are each independently integers from 0 to 3,
q is 0 or 1,
R^{f}, R^{g}, R^{h}, Rⁱ, R^{j} and R^{k} independently represent hydrogen atoms or halogen atoms,
R^{b'} is selected from the group consisting of methylsulphonyl groups, C₁₋₄ alkyl groups, C₁₋₄ hydroxyalkyl groups, C₁₋₄ carboxyalkyl groups, and C₁₋₄ haloalkyl groups;
R^{a} and R^{b} are independently selected from the group consisting of hydrogen atoms, methylsulphonyl groups, C₁₋₄ alkyl groups, C₁₋₄ hydroxyalkyl groups, C₁₋₄ carboxyalkyl groups, and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by - NHR' wherein R' represents a hydrogen atom or a 6₁₋₄ carboxyalkyl group.

## Description

The present invention relates to new compounds, in particular new tetrahidroisoquinolyl derivatives, to processes for their preparation and to pharmaceutical compositions containing them. These compounds are potent agonists of S1P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

Sphingosine -1 phosphate (S1P) is a pleiotropic lipid mediator that exhibits a broad spectrum of biological activities, including cell proliferation, survival, lymphocyte trafficking, cytoskeletal organization, and morphogenesis. S1P is generated from endogenous sphingosine through phosphorylation by specific kinases, named sphingosine kinases 1 and 2. The levels of S1P in biological fluids and tissues are tightly regulated by the balance between its synthesis by sphingosine kinases and its degradation by S1P lyase. This tight control is important since an excessive production of S1P has been associated to various pathological conditions, such as angiogenesis and vascular permeability changes in cancer, inflammation, myocardial infarction or transplant rejection.

Gene deletion studies and reverse pharmacology have provided evidence that most of the effects of S1P are mediated via five G-protein coupled receptor subtypes, named S1P1 to S1P5 (Brinkmann, Pharmacology & therapeutics 115:84-105, 2007). The interest on this family of receptors increased following the discovery that they were the pharmacological target of FTY720. This compound, a synthetic analog of a natural product derived from the fungus *Isaria sinclairii,* exhibited a peculiar immunomodulatory potential in vivo. When administered in vivo, it caused lymphopenia, due to the sequestration of lymphocytes from the blood into the lymph nodes and Peyer's patches. The close structural similarity of FTY720 to sphingosine, together with the discovery of the formation of phosphorylated FTY720 in vivo (FTY720P) prompted to speculate that FTY720-P could be acting as a mimetic of S1P. This proven to be the case and it was later on demonstrated that FTY-P binds 4 of the five known S1P receptors, namely S1P1, S1P3, S1P4 and S1P5.

Expression analysis identified S1P1 as the dominant S1P receptor expressed on lymphocytes. Moreover, the transfer of S1P1-deficient T cells to normal mice led to the cells being sequestered in lymph nodes, as occurred with animals treated with fingolimod. These two facts strongly pointed out at S1P1 as the main receptor involved in the lymphopenic effect of FTY-P in vivo (Baumruker et al, Exp. Opin. Invest. Drugs 2007; 16(3): 283-289). FTY720 is currently in phase III trials for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943) cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295), cardiovascular diseases (WO2004010987),.

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes; systemic lupus erythematosis and Sjögrn's syndrome.

Transplant rejections include but are not limited to rejections of kidney, liver, heart, lung, pancreas, cornea and skin transplants and graft-versus-host disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies.

Angiogenesis-related disorders that may be prevented or treated include but are not limited to hemangiomas, ocular neovascularization, macular degeneration or diabetic retinopathy.

Pain, including neuropathic pain, that may be prevented or treated includes but is not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation, trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction and complications from cardiac surgery. Cardiovascular diseases may also refer to improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain tetrahidroisoquinolyls derivatives are novel and potent agonists of S1P1 and can therefore be used in the treatment or prevention of these diseases.

Thus the present invention is directed to new tetrahidroisoquinolyls derivatives of formula (I) or pharmaceutically acceptable salts or N-oxides thereof 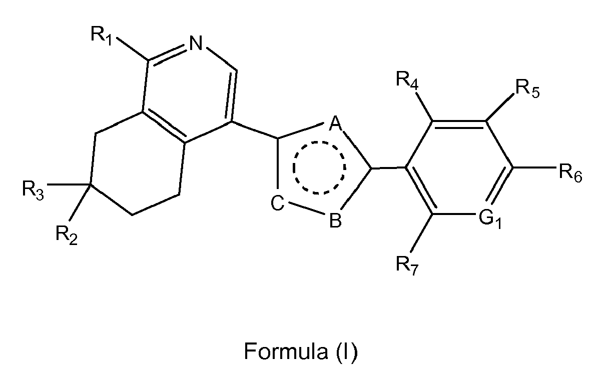 wherein
A is selected from the group consisting of -N-, -O- and -S-;
B and C independently are selected from the group consisting of -N- and -O-, with the proviso that at least two of A, B and C are nitrogen atoms;
G¹ is selected from the group consisting of nitrogen atoms and -CR^{c}- groups, wherein R^{c} represents a hydrogen atom, a halogen atom, a C₁₋₄ alkyl group or a C₁₋₄ alkoxy group;
R¹ is selected from the group consisting of hydrogen atoms, C₁₋₄ alkyl groups, C₁₋₄ alkoxy groups, C₃₋₄ cycloalkyl groups, and -NR^{d}R^{e} groups wherein R^{d} and R^{e} are independently selected from hydrogen atoms and C₁₋₄ alkyl groups;
R² and R³ are independently selected from the group consisting of hydrogen atoms and C₁₋₄ alkyl groups;
R⁴, R⁵ and R⁷ are independently selected from the group consisting of hydrogen atoms, halogen atoms, C₁₋₄ alkyl groups, C₁₋₄ alkoxy groups and C₁₋₄ haloalkyl groups;
R⁶ represents a C₁₋₄ alkyl group or a C₁₋₄ hydroxyalkyl group; or R⁶ is selected from the group consisting of -S(O)₂-NR^{a}R^{b} groups, -(CR^{f}R^{g})ₙ-(CR^{h}Rⁱ)_{X}-(CR^{j}R^{k})_{y}-NR^{a}R^{b} groups,
-(CH₂)ₙ-NR^{a}R^{b} groups, -O-(CH₂)ₙ-NR^{a}R^{b} groups, -(CH₂)ₙ-COOH groups, -(CH₂)ₙ-NR^{a}-CO-R^{b'} groups, -(CH₂)ₙ-NR^{a}-(CH₂)ₚ-(NH)_{q}-SO-CH₃ groups and -(CH₂)ₙ-CO-NR^{a}R^{b} groups, wherein
n, p, x and y are each independently integers from 0 to 3;
q is 0 or 1;
R^{f}, R^{g}, R^{h}, Rⁱ, R^{j} and R^{k} independently represent hydrogen atoms or halogen atoms;
R^{b'} is selected from the group consisting of methylsulphonyl groups, C₁₋₄ alkyl groups, C₁₋₄ hydroxyalkyl groups, C₁₋₄ carboxyalkyl groups, and C₁₋₄ haloalkyl groups;
R^{a} and R^{b} are independently selected from the group consisting of hydrogen atoms, methylsulphonyl groups, C₁₋₄ alkyl groups, C₁₋₄ hydroxyalkyl groups, C₁₋₄ carboxyalkyl groups, and C₁₋₄ haloalkyl groups, or
R^{a} and R^{b} or R^{a} and R^{b'} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by -NHR' wherein R' represents a hydrogen atom or a C₁₋₄ carboxyalkyl group.

Further objectives of the present invention are to provide a method for preparing said compounds; pharmaceutical compositions comprising an effective amount of said compounds; the use of the compounds in the manufacture of a medicament for the treatment of pathological conditions or diseases susceptible to improvement by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases, and methods of treatment of pathological conditions or diseases susceptible to amelioration by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases comprising the administration of a therapeutically effective amount of the compounds of the invention to a subject in need of such treatment.

As used herein the term C₁₋₄ alkyl embraces optionally substituted, linear or branched hydrocarbon radicals having 1 to 4 carbon atoms. Preferred substituents on the alkyl groups are halogen atoms and hydroxy groups, and are more preferably halogen atoms. Examples include methyl, ethyl, *n*-propyl, *i*-propyl, *n*-butyl, *i*-butyl, *sec*-butyl and *tert*-butyl radicals.

As used herein, the term C₁₋₄ haloalkyl group is an alkyl group, for example a C₁₋₄ or C₁₋₂ alkyl group, which is attached to 1, 2 or 3 halogen atoms. Preferably, said C₁₋₄ haloakyl group is chosen from -C(-)Cl-, -CHCl-, -CCl₂-, -CHCl₂, -CCl₃, -C(-)F-, -CHF-, -CF₂-, -CHF₂, -CF₃ and -CH₂CHF₂. More preferably, said C₁₋₄ haloalkyl group is chosen from -CH₂Cl, -CHCl₂, -CCl₃, -CH₂F, -CHF₂, -CF₃ and -CH₂CHF₂, most preferably - CH₂CHF₂.

As used herein, the term C₁₋₄ hydroxyalkyl embraces linear or branched alkyl radicals having 1 to 4 carbon atoms, any one of which may be substituted with one or more hydroxyl radicals. Examples of such radicals include hydroxymethyl, hydroxyethyl, hydroxypropyl, hydroxybutyl, dihydroxyethyl, 1,3-dihydroxypropyl, 2,3-dihydroxypropyl, 1,3-dihydroxy isopropyl, 2,3-dihydroxybutyl and 3,4-dihydroxybutyl radicals.

As used herein, the term C₁₋₄ alkoxy (or alkyloxy) embraces optionally substituted, linear or branched oxy-containing radicals each having alkyl portions of 1 to 4 carbon atoms. More preferred alkoxy radicals are alkoxy radicals having 1 to 2 carbon atoms. An alkoxy group is typically unsubstituted or substituted with 1, 2 or 3 substituents which may be the same or different. The substituents are preferably selected from halogen atoms, preferably chlorine or fluorine atoms and hydroxy groups. Typically, the substituents on an alkoxy group are themselves unsubstituted. Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, the term carboxyl embraces a -COOH group.

As used herein, the term C₁₋₄ carboxyalkyl group embraces optionally substituted, linear or branched carboxyl-containing radicals having 1 to 4 carbon atoms. Preferred substituents on the alkyl group of the carboxyalkyl groups are halogen atoms and hydroxy groups.

As used herein, the term cycloalkyl embraces saturated carbocyclic radicals and, unless otherwise specified, a cycloalkyl radical typically has from 3 to 6 carbon atoms, preferably from 3 to 4 carbon atoms. Examples include cyclopropyl, cyclobutyl, cyclopentyl and cyclohexyl. When a cycloalkyl radical carries 2 or more substituents, the substituents may be the same or different. Preferred substituents on the cycloalkyl groups are halogen atoms and hydroxy groups, and are more preferably halogen atoms.

As used herein, the term heterocyclic group embraces typically a non-aromatic, saturated or unsaturated 4 to 6 membered ring system, in which one or more, for example 1, 2, or 3 of the carbon atoms preferably 1 or 2 of the carbon atoms are replaced by nitrogen. Saturated heterocyclyl radicals are preferred. A heterocyclic radical may be a single ring or two or more fused rings wherein at least one ring contains a heteroatom. When a heterocyclyl radical carries 2 or more substituents, the substituents may be the same or different.

A said optionally substituted heterocyclic group is typically unsubstituted or substituted with 1 or 2 substituents which may be the same or different. The substituents are preferably a carboxyl group or a C₁₋₄ carboxyalkyl group. Typically, the substituents on a heterocyclyl group are themselves unsubstituted. Where the heterocyclic group carries 2 or more substituents, the substituents may be the same or different.

Examples of heterocyclic radicals include azetidyl, piperidyl, pyrrolidyl, pyrrolinyl, piperazinyl, pyrazolinyl and pyrazolidinyl. Azetidyl and piperazinyl are preferred.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles.

When two or more substituents are present, each substituent may be the same or different.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

Preferably, when R^{a} and R^{b} together with the nitrogen atom to which they are attached do not form a 4 to 6 membered, saturated heterocyclic group, which contains, as heteroatoms, one or two nitrogen atoms and which is substituted by a carboxyl group or a C₁₋₄ carboxyalkyl group, then either (a) R^{a} and R^{b} are independently selected from the group consisting of hydrogen atoms, methylsulphonyl groups, C₁₋₄ alkyl groups, C₁₋₄ hydroxyalkyl groups, and C₁₋₄ carboxyalkyl groups, or (b) R^{a} and R^{b} each independently represents a C₁₋₄ alkyl group or a C₁₋₄ haloalkyl group.

Typically, in the compound of formula (I), A is selected from the group consisting of -N- and -O-. Preferably, A represents -N-.

The dotted line in the ring containing A, B and C denotes that the ring may be saturated, unsaturated or aromatic. Preferably, the ring is aromatic, i.e. is represented by the formula

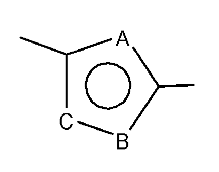

Typically, in groups of formula -S(O)₂-NR^{a}R^{b}, -(CR^{f}R^{g})ₙ-(CR^{h}Rⁱ)_{X}-(CR^{j}R^{k})_{y}-NR^{a}R^{b}, -(CH₂)ₙ-NR^{a}R^{b}, -O-(CH₂)ₙ-NR^{a}R^{b}, and -(CH₂)ₙ-CO-NR^{a}R^{b}, R^{a} and R^{b} are not simultaneously methylsulphonyl groups.

More typically, in groups of formula -S(O)₂-NR^{a}R^{b}, -(CR^{f}R^{g})ₙ-(CR^{h}Rⁱ)_{X}-(CR^{j}R^{k})_{y}-NR^{a}R^{b}, -(CH₂)ₙ-NR^{a}R^{b}, -O-(CH₂)ₙ-NR^{a}R^{b}, and -(CH₂)ₙ-CO-NR^{a}R^{b}, R^{a} is hydrogen and R^{b} is selected from the group consisting of hydrogen atoms, methylsulphonyl groups, C₁₋₄ alkyl groups, C₁₋₄ hydroxyalkyl groups, C₁₋₄ carboxyalkyl groups, and C₁₋₄ haloalkyl groups, or R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group.

Preferably, in groups of formula -S(O)₂-NR^{a}R^{b}, -(CR^{f}R^{g})ₙ-(CR^{h}Rⁱ)_{X}-(CR^{j}R^{k})_{y}-NR^{a}R^{b}, -(CH₂)ₙ-NR^{a}R^{b}, -O-(CH₂)ₙ-NR^{a}R^{b}, and -(CH₂)ₙ-CO-NR^{a}R^{b}, R^{a} is hydrogen and R^{b} is selected from the group consisting of hydrogen atoms, methylsulphonyl groups, C₁₋₄ alkyl groups, C₁₋₄ carboxyalkyl groups, and C₁₋₄ haloalkyl groups, or R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group.

More preferably, in groups of formula -S(O)₂-NR^{a}R^{b}, -(CR^{f}R^{g})ₙ-(CR^{h}Rⁱ)_{X}-(CR^{j}R^{k})_{y}-NR^{a}R^{b}, -(CH₂)ₙ-NR^{a}R^{b}, -O-(CH₂)ₙ-NR^{a}R^{b}, and -(CH₂)ₙ-CO-NR^{a}R^{b}, R^{a} is hydrogen and R^{b} is selected from the group consisting of hydrogen atoms, methylsulphonyl groups, C₁₋₂ carboxyalkyl groups, and C₁₋₂ haloalkyl groups, or R^{a} and R^{b} together with the nitrogen atom to which they are attached form an azetidine or piperazine group which groups are substituted by a carboxyl group or a C₁₋₂ carboxyalkyl group.

Typically, in the compound of formula (I), G¹ represents a -CR^{c}- group, wherein R^{c} represents a hydrogen atom, chlorine atom, fluorine atom or a C₁₋₄ alkyl group. Preferably, G¹ represents a -CR^{c}- group, wherein R^{c} represents a hydrogen atom or a methyl group.

Typically, in the compound of formula (I), R¹ represents a hydrogen atom, a C₁₋₄ alkyl group or a C₃₋₄ cycloalkyl group. Preferably, R¹ is selected from the group consisting of hydrogen atoms, ethyl groups and C₃₋₄ cycloalkyl groups.

Typically, R² represents a hydrogen atom or a C₁₋₄ alkyl group, preferably R² represents a hydrogen atom or a methyl group. More typically R² represents a C₁₋₄ alkyl group.

Typically, R³ represents a hydrogen atom or a C₁₋₄ alkyl group, preferably R³ represents a hydrogen atom or a methyl group. More typically R³ represents a C₁₋₄ alkyl group.

Typically, R⁴ represents a hydrogen atom, a C₁₋₄ alkyl group or a -CF₃ group, preferably a hydrogen atom or a methyl group, more preferably a hydrogen atom.

Typically, R⁵ represents a hydrogen atom, a C₁₋₄ alkyl group or a -CF₃ group, preferably a hydrogen atom or a C₁₋₄ alkyl group, more preferably a hydrogen atom or a methyl group, being the most preferred a methyl group.

Typically, R⁷ represents a hydrogen atom, a C₁₋₄ alkyl group or a -CF₃ group, preferably a hydrogen atom or a methyl group, more preferably a hydrogen atom.

Typically, at least one of R⁴, R⁵ and R⁷ is a hydrogen atom. More typically, 1 or 2 of R⁴, R⁵ and R⁷ are hydrogen atoms. Preferably, two of R⁴, R⁵ and R⁷ are hydrogen atoms. More preferably, two of R⁴, R⁵ and R⁷ are hydrogen atoms and the other is methyl. Even more preferably, R⁴, and R⁷ are hydrogen atoms and R⁵ is methyl.

Typically, R⁶ represents a C₁₋₄ alkyl group, or a C₁₋₄ hydroxyalkyl group; or R⁶ is selected from the group consisting of -(CH₂)ₙ-NR^{a}R^{b} groups, -(CH₂)ₙ-COOH groups, -(CH₂)ₙ-NR^{a}-(CH₂)ₚ-(NH)_{q}-SO-CH₃ groups and -(CH₂)ₙ-CO-NR^{a}R^{b} groups, wherein
n and p are each independently integers from 0 to 3,
q is 0 or 1,
R^{a} and R^{b} are independently selected from the group consisting of hydrogen atoms, methylsulphonyl groups, C₁₋₄ carboxyalkyl groups, C₁₋₄ alkyl groups and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group.
In a particularly preferred embodiment, in the compound of formula (I)
A represents -N-;
G¹ represents a -CR^{c}- group, wherein R^{c} represents a hydrogen atom or C₁₋₄ alkyl group;
R² and R³ independently represent C₁₋₄ alkyl groups;
R⁴, R⁵ and R⁷ are independently selected from the group consisting of hydrogen atoms and C₁₋₄ alkyl groups;
R⁶ represents a C₁₋₄ alkyl group or a C₁₋₄ hydroxyalkyl group, or R⁶ is selected from the group consisting of -S(O)₂-NR^{a}R^{b} groups, -(CH₂)ₙ-NR^{a}R^{b} groups, -(CH₂)ₙ-COOH groups, -(CH₂)ₙ-NR^{a}-(CH₂)ₚ-(NH)_{q}-SO-CH₃ groups and -(CH₂)ₙ-CO-NR^{a}R^{b} groups, wherein
n and p are each independently integers from 0 to 3,
q is 0 or 1,
R^{a} and R^{b} independently are selected from the group consisting of hydrogen atoms, methylsulphonyl groups, C₁₋₄ carboxyalkyl groups, C₁₋₄ alkyl groups and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by -NHR' wherein R' represents a hydrogen atom or a C₁₋₄ carboxyalkyl group.

In a further particularly preferred embodiment, compounds compounds of the present invention are compounds of formula (I'), or pharmaceutically acceptable salts or N-oxides thereof, 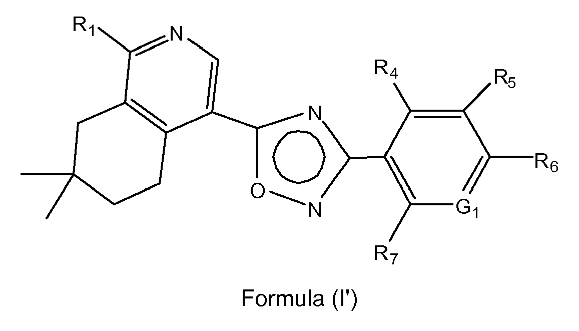 wherein
G¹ is selected from the group consisting of nitrogen atoms and -CR^{c}- groups, wherein R^{c} represents a hydrogen atom, a chlorine atom, or a C₁₋₂ alkyl group;
R¹ is selected from the group consisting of hydrogen atoms, C₁₋₂ alkyl groups, cyclopropyl groups, and -NH₂ groups;
R⁴, R⁵ and R⁷ are independently selected from the group consisting of hydrogen atoms, chlorine atoms, C₁₋₂ alkyl groups;
R⁶ represents a C₁₋₂ alkyl group or a C₁₋₄ hydroxyalkyl group; or R⁶ is selected from the group consisting of -S(O)₂-NHR^{b} groups, -(CH₂)ₙ-NHR^{b} groups, -(CH₂)ₙ-COOH groups, and -(CH₂)ₙ-CO-NHR^{b} groups, wherein
n is 2 or 3,
R^{b} is selected from the group consisting of hydrogen atoms, methylsulphonyl groups, C₁₋₂ carboxyalkyl groups, and C₁₋₂ haloalkyl groups, or
R^{a} and R^{b} together with the nitrogen atom to which they are attached form an azetidine or piperazine group which groups are substituted by a carboxyl group or a C₁₋₂ carboxyalkyl group; or
R^{c} together with R⁶ form a C6 carbocyclic ring substituted by -NHR' wherein R' represents a hydrogen atom or a C₁₋₂ carboxyalkyl group.
In a further particularly preferred embodiment, R⁶ represents a C₁₋₄ alkyl group, or a C₁₋₄ hydroxyalkyl group, or
R⁶ is selected from the group consisting of -S(O)₂-NR^{a}R^{b} groups, -(CH₂)ₙ-NR^{a}R^{b} groups, -(CH₂)ₙ-COOH groups, and -(CH₂)ₙ-CO-NR^{a}R^{b} groups, wherein,
n is an integer from 0 to 3,
R^{a} and R^{b} are independently selected from the group consisting of hydrogen atoms, methylsulphonyl groups and C₁₋₄ carboxyalkyl groups, C₁₋₄ alkyl groups and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by -NHR' wherein R' represents a hydrogen atom or a C₁₋₄ carboxyalkyl group.

Particular individual compounds of the invention include:
(4-(5-(1-ethyl-7,7-dimethyl-5,6,7,8-tetrahydroisoquinolin-4-yl)-1,2,4-oxadiazol-3-yl)phenyl)methanol
4-(5-(1-ethyl-7,7-dimethyl-5,6,7,8-tetrahydroisoquinolin-4-yl)-1,2,4-oxadiazol-3-yl)benzenesulfonamide
3-(4-(4-(5-(1-ethyl-7,7-dimethyl-5,6,7,8-tetrahydroisoquinolin-4-yl)-1,2,4-oxadiazol-3-yl)phenyl)piperazin-1-yl)propanoic acid
3-(4-(5-(1-ethyl-7,7-dimethyl-5,6,7,8-tetrahydroisoquinolin-4-yl)-1,2,4-oxadiazol-3-yl)phenylsulfonamido)propanoic acid
3-(4-(5-(1-ethyl-7,7-dimethyl-5,6,7,8-tetrahydroisoquinolin-4-yl)-1,2,4-oxadiazol-3-yl)-2,6-dimethylphenyl)propanoic acid
3-(4-(5-(1-ethyl-7,7-dimethyl-5,6,7,8-tetrahydroisoquinolin-4-yl)-1,2,4-oxadiazol-3-yl)-2,6-dimethylphenyl)propanamide
2-(4-(5-(1-ethyl-7,7-dimethyl-5,6,7,8-tetrahydroisoquinolin-4-yl)-1,2,4-oxadiazol-3-yl)-2,6-dimethylphenyl)ethanamine
3-(4-(5-(1-cyclopropyl-7,7-dimethyl-5,6,7,8-tetrahydroisoquinolin-4-yl)-1,2,4-oxadiazol-3-yl)-2,6-dimethylphenyl)propanoic acid
3-(4-(5-(7,7-dimethyl-5,6,7,8-tetrahydroisoquinolin-4-yl)-1,2,4-oxadiazol-3-yl)-2,6-dimethylphenyl)propanoic acid
2-(4-(5-(1-cyclopropyl-7,7-dimethyl-5,6,7,8-tetrahydroisoquinolin-4-yl)-1,2,4-oxadiazol-3-yl)-2,6-dimethylphenyl)ethanamine
2-(4-(5-(7,7-dimethyl-5,6,7,8-tetrahydroisoquinolin-4-yl)-1,2,4-oxadiazol-3-yl)-2,6-dimethylphenyl)ethanamine
3-(4-(5-(7,7-dimethyl-5,6,7,8-tetrahydroisoquinolin-4-yl)-1,2,4-oxadiazol-3-yl)-3-methylphenyl)propanoic acid
2-(4-(5-(7,7-dimethyl-5,6,7,8-tetrahydroisoquinolin-4-yl)-1,2,4-oxadiazol-3-yl)-3-methylphenyl)ethanamine
2-(3-(4-(5-(7,7-dimethyl-5,6,7,8-tetrahydroisoquinolin-4-yl)-1,2,4-oxadiazol-3-yl)-2,6-dimethylphenyl)propanamido)ethanoic acid
3-(4-(5-(7,7-dimethyl-5,6,7,8-tetrahydroisoquinolin-4-yl)-1,2,4-oxadiazol-3-yl)phenyl)propanoic acid
4-(4-(5-(7,7-dimethyl-5,6,7,8-tetrahydroisoquinolin-4-yl)-1,2,4-oxadiazol-3-yl)phenyl)butanoic acid
2-(4-(5-(7,7-dimethyl-5,6,7,8-tetrahydroisoquinolin-4-yl)-1,2,4-oxadiazol-3-yl)phenyl)ethanamine
6-(5-(7,7-dimethyl-5,6,7,8-tetrahydroisoquinolin-4-yl)-1,2,4-oxadiazol-3-yl)-1,2,3,4-tetrahydronaphthalen-2-amine
3-(4-(5-(7,7-dimethyl-5,6,7,8-tetrahydroisoquinolin-4-yl)-1,2,4-oxadiazol-3-yl)-2,6-dimethylphenyl)propanamide
2-(6-(5-(7,7-dimethyl-5,6,7,8-tetrahydroisoquinolin-4-yl)-1,2,4-oxadiazol-3-yl)-1,2,3,4-tetrahydronaphthalen-2-ylamino)ethanoic acid
3-(4-(5-(7,7-dimethyl-5,6,7,8-tetrahydroisoquinolin-4-yl)-1,2,4-oxadiazol-3-yl)-2,6-dimethylphenyl)propan-1-amine
3-(4-(5-(7,7-dimethyl-5,6,7,8-tetrahydroisoquinolin-4-yl)-1,2,4-oxadiazol-3-yl)-2,6-dimethylphenyl)propane-1,2-diol
N-(3-(4-(5-(7,7-dimethyl-5,6,7,8-tetrahydroisoquinolin-4-yl)-1,2,4-oxadiazol-3-yl)-2,6-dimethylphenyl)propyl)methanesulfonamide
N-(4-(5-(7,7-dimethyl-5,6,7,8-tetrahydroisoquinolin-4-yl)-1,2,4-oxadiazol-3-yl)-2,6-dimethylphenethyl)-2,2-difluoroethanamine
1-(4-(5-(7,7-dimethyl-5,6,7,8-tetrahydroisoquinolin-4-yl)-1,2,4-oxadiazol-3-yl)-2,6-dimethylphenethyl)azetidine-3-carboxylic acid
3-(5-(5-(7,7-dimethyl-5,6,7,8-tetrahydroisoquinolin-4-yl)-1,2,4-oxadiazol-3-yl)-3-ethyl-6-methylpyridin-2-yl)propanoic acid
3-(2-chloro-4-(5-(7,7-dimethyl-5,6,7,8-tetrahydroisoquinolin-4-yl)-1,2,4-oxadiazol-3-yl)phenylsulfonamido)propanoic acid
3-(4-(5-(7,7-dimethyl-5,6,7,8-tetrahydroisoquinolin-4-yl)-1,2,4-oxadiazol-3-yl)phenylsulfonamido)propanoic acid
3-(4-(5-(1-amino-7,7-dimethyl-5,6,7,8-tetrahydroisoquinolin-4-yl)-1,2,4-oxadiazol-3-yl)-2,6-dimethylphenyl)propane-1,2-diol
4-(4-(5-(7,7-dimethyl-5,6,7,8-tetrahydroisoquinolin-4-yl)-1,2,4-oxadiazol-3-yl)-2,6-dimethylphenyl)butanoic acid
1-(4-(5-(1-amino-7,7-dimethyl-5,6,7,8-tetrahydroisoquinolin-4-yl)-1,2,4-oxadiazol-3-yl)-2,6-dimethylphenethyl)azetidine-3-carboxylic acid

Of outstanding interest are:
3-(4-(5-(1-ethyl-7,7-dimethyl-5,6,7,8-tetrahydroisoquinolin-4-yl)-1,2,4-oxadiazol-3-yl)-2,6-dimethylphenyl)propanamide
2-(4-(5-(1-ethyl-7,7-dimethyl-5,6,7,8-tetrahydroisoquinolin-4-yl)-1,2,4-oxadiazol-3-yl)-2,6-dimethylphenyl)ethanamine
3-(4-(5-(1-cyclopropyl-7,7-dimethyl-5,6,7,8-tetrahydroisoquinolin-4-yl)-1,2,4-oxadiazol-3-yl)-2,6-dimethylphenyl)propanoic acid
2-(4-(5-(1-cyclopropyl-7,7-dimethyl-5,6,7,8-tetrahydroisoquinolin-4-yl)-1,2,4-oxadiazol-3-yl)-2,6-dimethylphenyl)ethanamine
2-(4-(5-(7,7-dimethyl-5,6,7,8-tetrahydroisoquinolin-4-yl)-1,2,4-oxadiazol-3-yl)-2,6-dimethylphenyl)ethanamine
3-(4-(5-(7,7-dimethyl-5,6,7,8-tetrahydroisoquinolin-4-yl)-1,2,4-oxadiazol-3-yl)-2,6-dimethylphenyl)propanamide
3-(4-(5-(7,7-dimethyl-5,6,7,8-tetrahydroisoquinolin-4-yl)-1,2,4-oxadiazol-3-yl)-2,6-dimethylphenyl)propan-1-amine
3-(4-(5-(7,7-dimethyl-5,6,7,8-tetrahydroisoquinolin-4-yl)-1,2,4-oxadiazol-3-yl)-2,6-dimethylphenyl)propane-1,2-diol
N-(4-(5-(7,7-dimethyl-5,6,7,8-tetrahydroisoquinolin-4-yl)-1,2,4-oxadiazol-3-yl)-2,6-dimethylphenethyl)-2,2-difluoroethanamine
1-(4-(5-(7,7-dimethyl-5,6,7,8-tetrahydroisoquinolin-4-yl)-1,2,4-oxadiazol-3-yl)-2,6-dimethylphenethyl)azetidine-3-carboxylic acid

Compounds of general formula (I) may be prepared following the synthetic scheme depicted in figure 1.

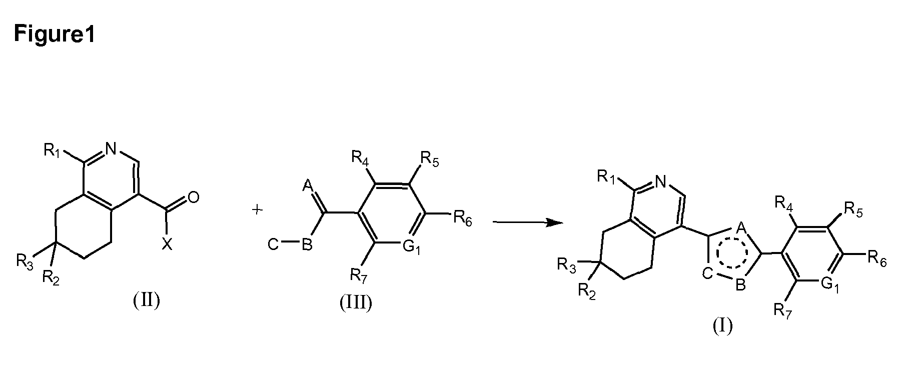

Thus, compounds of general formula (I) may be prepared by reacting a compound of general formula (II) wherein X can be OH or Cl, with a compound of structure (III) in a one pot reaction in solvent such as DMF, THF, etc., optionally in the presence of one or more coupling agents such as 2-(1H-benzotriazole-1-yl)-1,2,3,3-tetramethyluronium tetrafluoroborate (TBTU), N-(3-dimethylaminopropyl)-N'-ethyl-carbodiimide (EDC), dicyclohexyl carbodiimide (DCC), 1-hydroxybenzotriazole (HOBt), O-(benzotriazol-1-yl)-N,N,N',N'-tetramethyluroniium hexafluorophosphate (HBTU), carbonyl diimidazole (CDI), etc. and optionally in the presence of a base such as triethylamine, Hünig's base, sodium hydride, potassium carbonate, etc. at a temperature between 40 and 150°C and in a standard reactor. An alternative method could be following a two steps synthesis by a first coupling as described before and a subsequent cyclization in a solvent such as xylene, toluene, benzene, pyridine, DMF, dichloromethane, acetic acid, trifluoroacetic acid, etc. at rt or elevated temperatures optionally in the presence of auxiliaries such as acid (e.g. trifluoroacetic acid, acetic acid, hydrochloric acid, etc.), bases (e.g., sodium hydride, sodium acetate, sodium carbonate, potassium carbonate, triethylamine, etc.), tetralkylammonium salts or water removing agents such as oxalyl chloride, a carboxylic acid anhydride, phosphoryl trichloride (POCl₃), tetrabutylammonium fluoride (TBAF), molecular sieves, etc.

Alternatively compounds of general formula (I) may be prepared as shown in figure 2, by reacting an intermediate of general formula (IV) with an intermediate of formula (V) following the synthetic procedures described above.

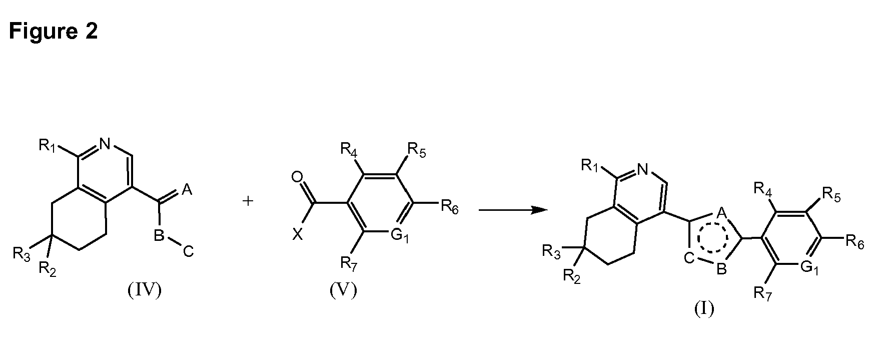

Intermediates of general formula (III) and (IV) may be prepared following the synthetic schemes depicted in figure 3.

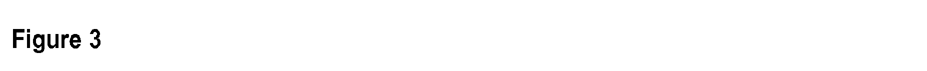

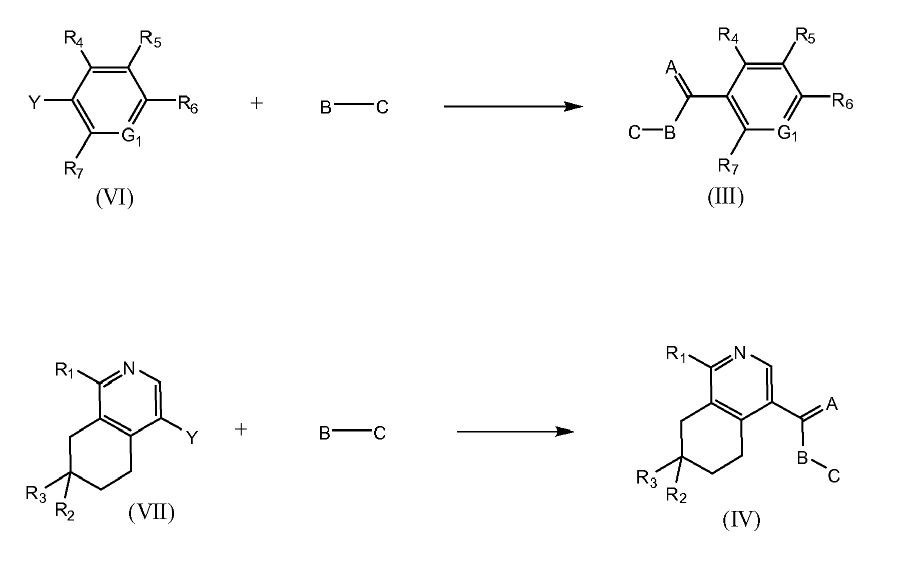

Intermediates of formula (III) and (IV) may be obtained from the corresponding intermediates of formula (VI) and (VII) wherein Y is CN, COOH, COCI or COOR' by reacting with hydroxylamine or hydrazine or one of its salts in a solvent such as THF, methanol, ethanol, pyridine, etc., optionally in the presence of a base such as sodium bicarbonate, sodium carbonate, potassium carbonate, triethylamine, sodium ethoxide etc. and at a temperature from room temperature to the boiling point of the solvent. Optionally, the reaction can be carried out in the presence of a coupling agent such as 2-(1H-benzotriazole-1-yl)-1,2,3,3-tetramethyluronium tetrafluoroborate, N-(3-dimethylaminopropyl)-N'-ethyl-carbodiimide, dicyclohexyl carbodiimide, 1-hydroxybenzotriazole, etc.

In the particular case of compounds of general formula (I) wherein A and B are nitrogen and C is an oxygen, compounds of general formula (Ia) may be prepared following the synthetic scheme depicted in figure 4.

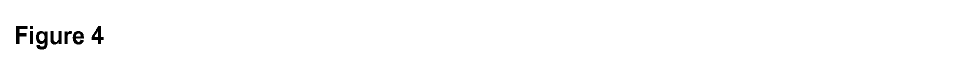

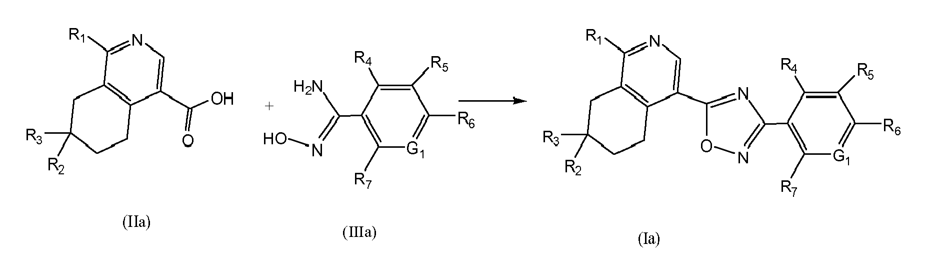

The oxadiazole compounds of general formula (Ia) may be prepared by the condensation of the tetrahidroisoquinoline-4-carboxylic acid (IIa) with the corresponding carboximidamide (IIIa) following the synthetic procedures described above.

Intermediates of general formula (IIa) may be prepared following the synthetic scheme depicted in figure 5.

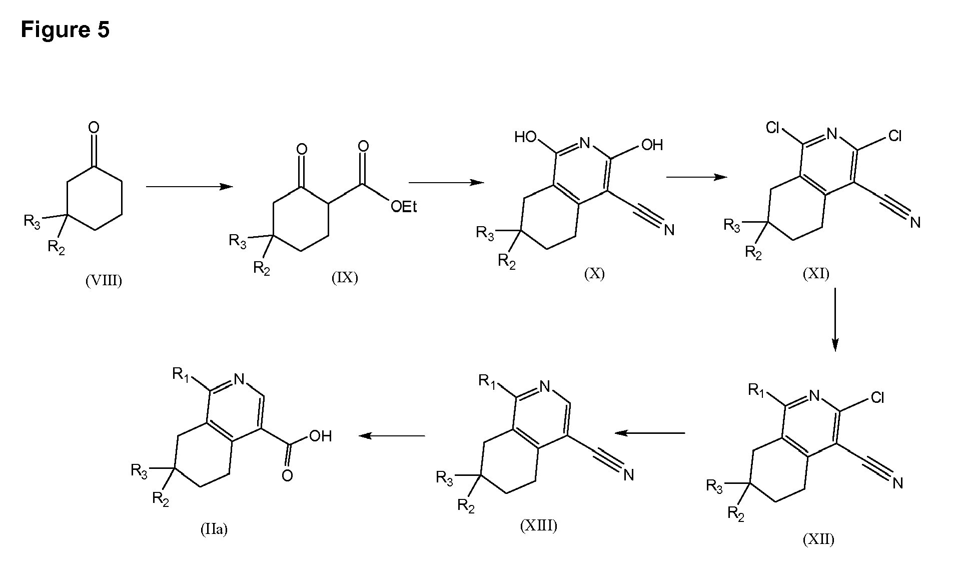

The intermediates of general formula (IX) may be prepared by the reaction of the corresponding commercial ketone (VIII) with diethylcarbonate in a basic media such as sodium hydride and in an aprotic solvent such as THF or DMF and at a temperature between 0 and the boiling point of the solvent.

The intermediates of general formula (X) may be prepared by the condensation of the intermediates of formula (IX) with 2-cyanoacetamide in basic media such as KOH or NaOH and in a protic solvent such as methanol or ethanol at a temperature between 20°C and the boiling point of the solvent.

The intermediates of general formula (XI) may be prepared by the reaction of the intermediates of formula (X) with POCl₃ or a mixture of POCl₃ and PCl₅ with or without a solvent such as toluene in a high pressure reactor and at high temperature.

The intermediates of general formula (XII) may be obtained by the coupling of the intermediates of formula (XI) with the corresponding alkyl boronic acid or boronate under the conditions of a Suzuki reaction (Miyaura, N.; Suzuki, A. Chem. Rev. 1995, 95, 2457). This reaction may be catalyzed by a palladium catalyst like [1,1'-bis(diphenylphosphino)-ferrocene] dichloropalladium (II) complex with dichloromethane (1:1), tetrakis(triphenylphosphine)-palladium(0), bis(triphenylphosphine)palladium(II) chloride or tris(dibenzylideneacetone)-dipalladium(0) in an aprotic organic solvent such as dioxane, toluene, DMF or DME and in the presence of a base such as cesium carbonate, sodium carbonate, potassium carbonate or potassium phosphate at a temperature from 80°C to 140°C.

In the particular case where R¹ is a -NR^{d}R^{e} group wherein R^{d} and R^{e} independently represent hydrogen atoms or a C₁₋₄ alkyl group and when R¹ is a C₁₋₄ hydroxyalkyl group, the intermediates of formula (XII) can be obtained by the reaction of the intermediates of formula (XI) with an excess of the corresponding amine or alcohol with an extra base such as NaH, triethylamine, etc. in a solvent such as MeOH, EtOH or THF and at temperature between 50 and 100°C.

Intermediates of general formula (XII) can also be obtained following the synthetic scheme shown in figure 6.

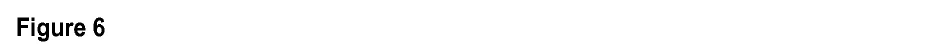

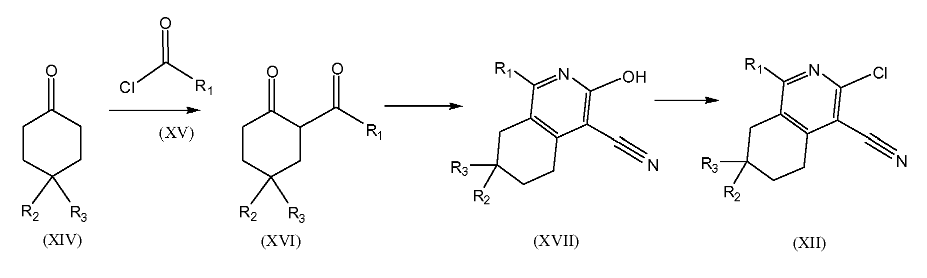

The intermediates of general formula (XVI) may be prepared by the reaction of the corresponding commercial ketone (XIV) with the corresponding acyl chloride of formula (XV) in a basic media such as lithiumdiisopropylamide (LDA) and in an aprotic solvent such as THF and at a temperature between -78°C and room temperature.

The intermediates of general formula (XVII) may be prepared by the condensation of the intermediates of formula (XVI) with 2-cyanoacetamide in basic media such as triethylamine, KOH or NaOH and in a protic solvent such as methanol or ethanol at a temperature between 20°C and the boiling point of the solvent.

Finally, intermediates of general formula (XII) may be prepared as intermediates of general formula (XI).

Reduction of intermediates of formula (XII) with a palladium catalyst such as Pd/C, palladium(II) chloride (PdCl₂) or dihydroxypalladium (Pd(OH)₂) in basic media such as sodium acetate, triethylamine (Et₃N) or diisopropylethylamine (DIEA) in a solvent such as methanol or ethanol under hydrogen atmosphere (from 1 to 20 psi), yield the tetrahydroisoquinoline intermediates of formula (XIII).

Hydrolysis of the nitrile compounds of general formula (XIII) with a base such as aqueous NaOH or KOH in a protic solvent such as methanol or ethanol at a temperature between 50 and 130°C or with an acid such as sulphuric acid, hydrochloric acid, in a solvent such as water, dioxane, acetic acid, etc. give the acid compound of formula (IIa).

Intermediates of general formula (IIIa) may be prepared following the synthetic scheme depicted in figure 7.

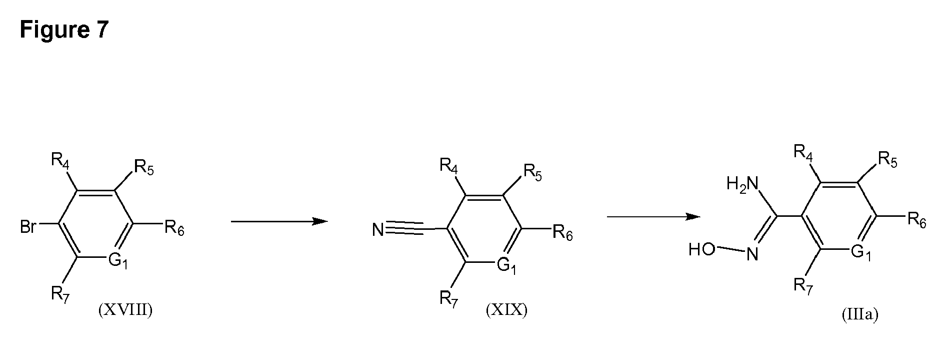

Intermediates of formula (IIIa) may be obtained by the reaction of hydroxylamine or one of its salts with the corresponding nitrile (XIX) in a solvent such as THF, methanol, ethanol, pyridine, etc. optionally in the presence of a base such as sodium bicarbonate, sodium carbonate, potassium carbonate, triethylamine, etc. and at a temperature from 40 to 100°C. The intermediates of structure (XIX) are commercially available or prepared according to procedures described in the art. A precursor can be the corresponding bromoaryl derivative of formula (XVIII) which react with a source of cyanide such as copper (I) cyanide, in a high boiling point solvent such as NMP, DMF or DMSO at a temperature between 150-200°C to yield the corresponding nitrile of general formula (XIV). Alternatively, dicyanozinc can be also used as a source of cyanide, with a Pd catalyst such as tetrakis(triphenylphosphine)-palladium(0) in a high boiling point solvent, in a standard reactor or a microwave reactor.
Depending on the nature of the functionalities present in the residues R₄ to R₇, these functionalities may require temporary protection. Appropriate protecting groups are known in the art and include e.g a *tert*-butyl or ethyl or methyl to protect an acid, a *tert-*butyloxycarbonyl (BOC) to protect an amine, etc. These protecting groups may be employed according to standard methodology (e.g. T.W. Greene, P. G. M. Wuts, Protective Groups in Organic Synthesis, 3rd Edition, Wiley New York, 1991).

In the particular case of intermediates of formula (IIIb) where R₆ is -(CH₂)₂COOR⁸ and R⁸ is a C₁₋₄ alkyl group, said intermediates may be obtained following the synthetic path shown in Figure 8.
Thus, a Heck reaction of the corresponding precursor (XX) wherein X= O-SO2-CF3, bromo or iodo, which are known or can be prepared according to known procedures, with acrylate yield the intermediates of formula (XXI). Reaction of these intermediates with hydroxylamine as described above and followed by catalytic hydrogenation, gave the intermediates of general formula (IIIb).

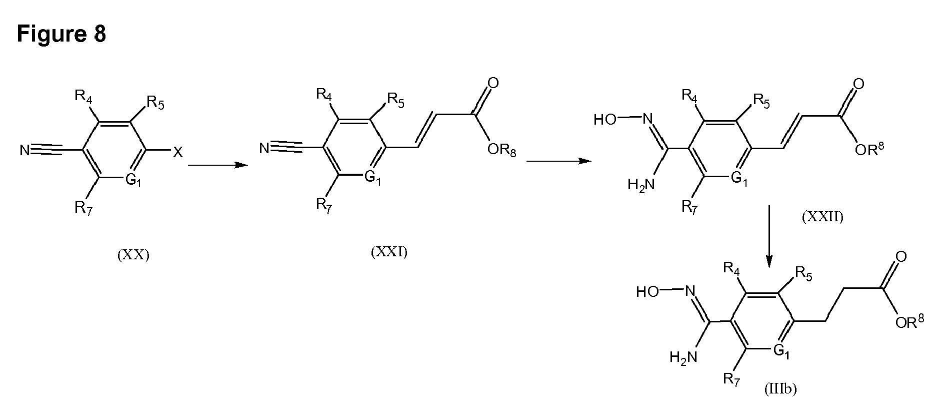

In the particular case of compounds of general formula (I) where R₆ is -(CH₂)ₙ-NR^{a}R^{b} group and a -(CH₂)ₙ-CO-NR^{a}R^{b} group these compounds can be obtained from compounds of general formula (Ib) wherein R₆ is a -(CH₂)ₙ-COOH group following the synthetic path shown in Figure 9.

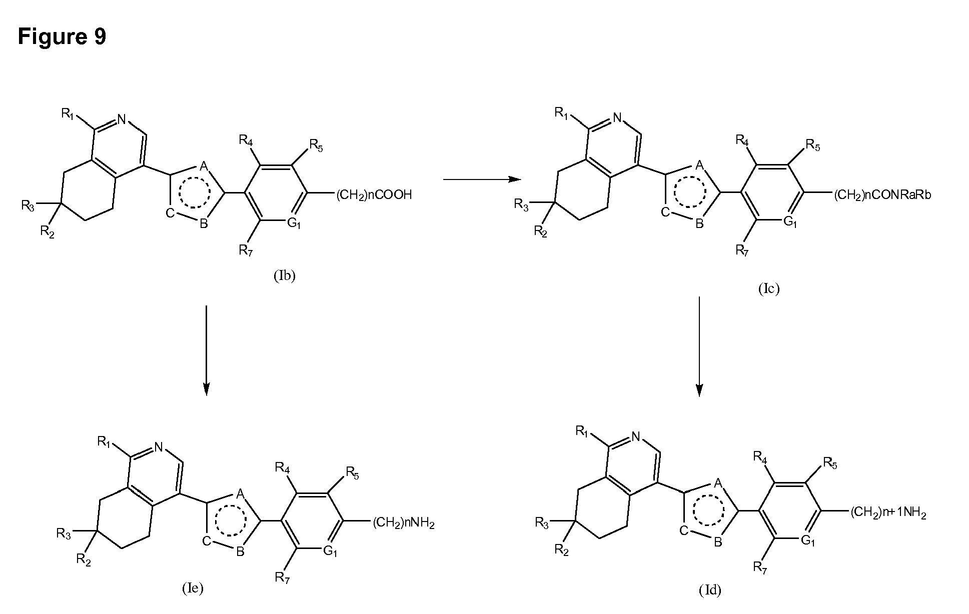

Thus, the compounds of general formula (Ic) wherein R₆ is a -(CH₂)ₙ-CO-NR^{a}R^{b} group can be prepared from the corresponding acid of general formula (Ib) by reacting with ammonia, an amine or an aminoacid of general formula HNR^{a}R^{b} in the presence of an activating agent such as N-(3-dimethylaminopropyl)-N'-ethyl-carbodiimide (EDC), dicyclohexyl carbodiimide (DCC), 1-hydroxybenzotriazole (HOBt), (benzotriazol-1-yloxy)-tris-(dimethylamino)-phosphonium hexafluorophosphate (BOP), bis-(2-oxo-3-oxazolidinyl)-phosphonic acid chloride (BOP-Cl), etc. in a solvent such as THF, dioxane, DMF, dichloromethane, acetonitrile, etc.

Reduction of primary amides of general formula (Ic) wherein R^{a}=R^{b}=H with BH₃.SMe₂ in a solvent such as THF, yield the corresponding primary amine of general formula (Id).

Moreover, primary amines of general formula (Ie) can be prepared by Curtius rearrangement of the acids of general formula (Ib) using an azide such as sodium azide, diphenylphosphoryl azide (DPPA), etc, in acidic media such as sulphuric acid or basic media such as triethylamine, in solvent such as toluene, chloroform, THF, etc. or in a protic solvent such as *tert*-butanol or benzyl alcohol to yield the *tert*-butylcarbonyl (BOC) or benzyloxycarbonyl (CBZ or Z) protected amine and subsequent deprotection as known in the art yield the final compounds of formula (Ie).

In the particular case where R₆ is -(CH₂)₂NHR^{a}R^{b} the compounds of formula (Ig) may be obtained following the synthetic path shown in Figure 10.

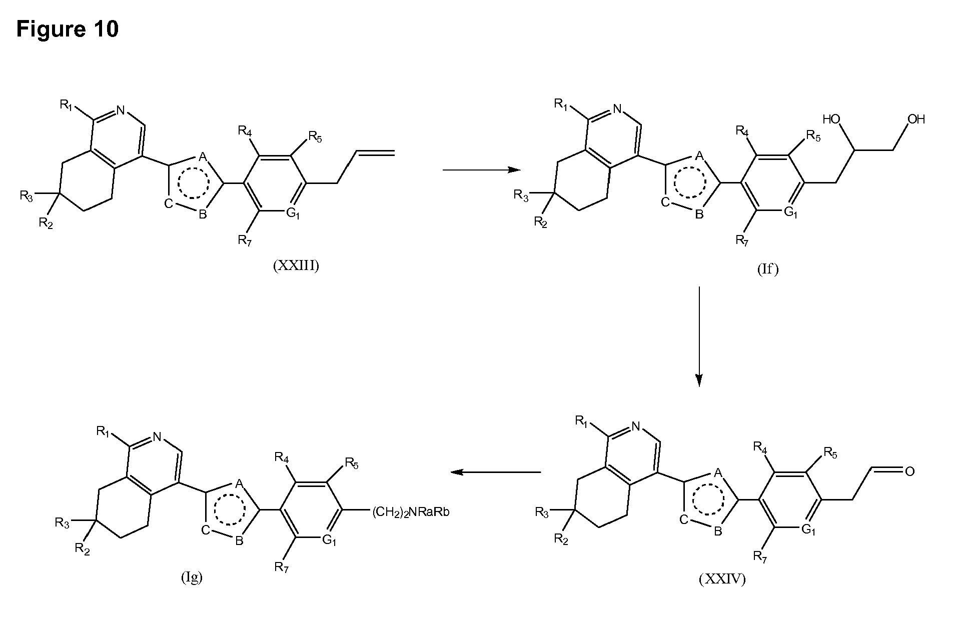

The compounds of general formula (Ig) may be prepared by the reductive amination of the aldehyde derivatives of general formula (XXIV) with the corresponding amine or aminoacid of formula HNR^{a}R^{b} in acidic media such as acetic acid, in a protic solvent such as methanol or ethanol and with a reductive agent such as sodium borohydride or sodium cyanoborohydride at a temperature from 0°C to the boiling point of the solvent.

Intermediates of formula (XXIV) may be obtained by oxidation of diols of general formula (If) with an oxidative reagent such as NalO₄, NaClO₄, KlO₄, etc. in a solvent such as methanol, ethanol, THF, dioxane, ether, etc. with the presence or absence of water.

Compounds of general formula (If) may be prepared by oxidation of the allyl intermediates of general formula (XXIII) using an oxidative reagent such as N-methylmorpholine-N-oxide, sodium periodate, sodium perchlorate, potassium periodate, etc. in the presence of a catalyst such us sodium tetraoxide solid or a solution in water or tert-butanol, in a solvent such as methanol, ethanol, THF, dioxane, ether, acetone, etc. with the presence or absence of water.

Allyl intermediates of general formula (XXIII) may be prepared by the condensation of the corresponding carboximidamide of formula (IIIc) with the corresponding acid of general formula (IIa) as described above.

Finally, as shown in figure 11, intermediates of formula (IIIc) wherein R₆ is - CH₂CH=CH₂ may be obtained by standard Stille reaction of the corresponding precursor (XX) where X= O-SO2-CF3, bromine or iodine, using an allylstannane and a catalyst such as tetrakis(triphenylphosphine)-palladium(0), palladium acetate, bis(triphenylphosphine)palladium(II) chloride or tris(dibenzylideneacetone)-dipalladium(0), in a solvent such DMF, acetonitrile, etc. and subsequent reaction with hydroxylamine as described before.

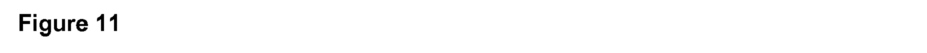

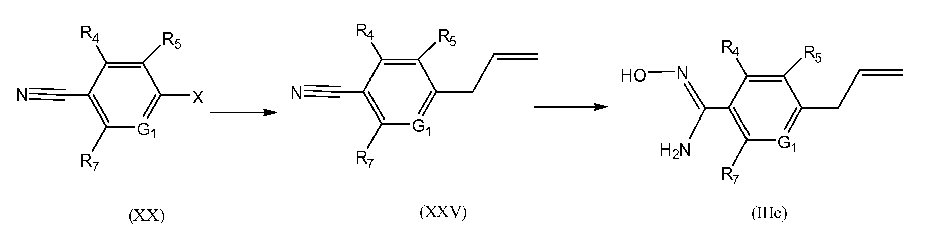

The syntheses of the compounds of the invention and of the Preparations for use therein are illustrated by the following Examples (1 to 31) including Preparation Examples (Preparations 1 to 75) which do not limit the scope of the invention in any way.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B and C. The injection volume was 5 microliter for method A and B and 3 microliter for method C. Diode array chromatograms were collected at 210 nM.

| Method | 0% B | 0 to 95% B | 95% B |
|---|---|---|---|
| A | 0.2 min | 3min | 0.8min |
| B | 0.5min | 6.5min | 1min |
| C | 0min | 20min | 4min |

### General purification method:

The solid was dissolved in DMSO/MeOH, injected into a Biotage C18 silica column (40M, 25M or 25S according to the crude amount) and eluted on the SP1® automated purification system from Biotage. The gradient used was H2O/Acetonitrile/MeOH (1:1) (0.1% v/v HCOONH4 both phases) from 0% to 100% acetonitrile/MeOH (1:1) in 80 column volumes. The appropriate fractions were collected and the organic solvent evaporated under reduced pressure or liofilized.

### GENERAL SYNTHETIC METHODS:

### General method 1:

A mixture of the corresponding benzonitrile (39.2mmol) in methanol (50mL), hydroxylamine hydrochloride (5.45g, 78.4mmol) and sodium bicarbonate (13.17g, 156.8mmol) was stirred at 75°C for 6h. The mixture was filtered off and the filtrate evaporated to dryness to yield the title compound (75-100% yield) as a white solid.

### General method 2:

A mixture of the corresponding acid derivative (1.13mmol), EDC (1.13mmol) and HOBt (1.13mmol) in DMF (5mL) was stirred at room temperature for 10 min. Then the corresponding benzimidamide (1.13mmol) was added and the mixture stirred at 140°C for 4h. The reaction mixture was poured over basic ice/water and the solid formed filtered and washed with water, dried in a vacuum oven to give the desired compound (40-90% yield) as a white solid.

### General method 3:

To a mixture of the corresponding methyl or ethyl esther (0.67mmol) in methanol or ethanol (3mL) respectively was added a 2M solution of aqueous NaOH (12mmol) and the reaction mixture stirred at 90°C overnight. The organic solvent was evaporated, water was added and the mixture extracted with diethyl ether. The pH of the aqueous layer was adjusted to 5-6 and the solid formed filtered and dried in the vacuum oven. The desired compound (60-95% yield) was obtained as a white solid.

### General method 4:

A mixture of the corresponding *tert*-butyl ester (0.56mmol) in 4M HCl in dioxane (3.5mL) was stirred at r.t. overnight. The solvent was evaporated and the solid obtained washed with diisopropyl ether twice. The solid was dried in the vacuum oven to yield (70-95% yield) of the desired compound.

### General method 5:

A mixture of the corresponding acid (0.46mmol), EDC (133mg, 0.69mmol), HOBt (94mg, 0.69mmol) and 32% solution of aqueous ammonia (85 µl, 0.69mmol) in DMF (6.5mL) was stirred overnight at room temperature. Then ethyl acetate and water were added and the organic layer separated, washed with 4% NaHCO3, water and brine and dried to give the title compound (5-77% yield) as a white solid.

### General method 6:

A mixture of the corresponding acid derivative (0.77mmol), DPPA (189µL, 0.88mmol) and triethylamine (235µL, 0.51mmol) in *tert*-butanol (4mL) was stirred at 100°C overnight. Ethyl acetate was added and the organic layer washed with 4% NaHCO3 and brine, dried and concentrated. The residue was redissolved in 4M HCl in dioxane (10mL) and the mixture stirred overnight at room temperature. The reaction mixture was concentrated and the residue purified according to General purification method. The solid obtained was redissolved in 4M HCl in dioxane and stirred for 2h at r.t. Then the solvent was concentrated and the product crystallized in diethyl ether. The title compound was obtained (5-40% yield) as hydrochloride salt.

### General method 7:

To a solution of the corresponding amide (75mg, 0.18mmol) in THF (4mL) was added BH₃.SMe₂ (107µL, 0.21 mmol) dropwise. The reaction mixture was stirred overnight at 65°C. Solvent was concentrated and ethyl acetate was added. The organic layer was washed with 4% NaHCO3 and brine, dried and concentrated. The residue was purified according to General purification method. The solid obtained was redissolved in 5N HCl in dioxane and stirred for 2h at r.t. Then the solvent was concentrated and the product crystallized in diethyl ether. The title compound was obtained (20-65% yield) as hydrochloride salt.

### PREPARATION EXAMPLES

### Preparation 1

### Ethyl 5,5-dimethyl-2-oxocyclohexanecarboxylate

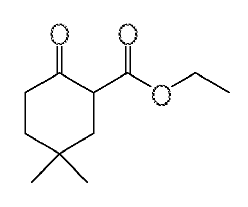

To a suspension of 60% NaH (74g, 1.85mol) in THF (1430mL) was added diethyl carbonate (269mL, 2.22mol) and the mixture was warm up to reflux. Then a solution of 4,4-dimethylcyclohexanone (93.44g, 0.74mol) in THF (1305mL) was added dropwise for 2.5h. The reaction mixture was stirred an extra 4h and then allowed to cool down to room temperature. Saturated solution of NH₄Cl was added carefully and the mixture stirred overnight. Organic layer was separated and the aqueous layer extracted with diethyl ether twice. The combined organic layer was washed with water and brine, dried over sodium sulphate and concentrated to yield 174g of an orange oil which was used without further purification.
LRMS: m/z 199 (M+1)⁺
Retention time: 6.10 min (Method B)

### Preparation 2

### 1,3-dihydroxy-7,7-dimethyl-5,6,7,8-tetrahydroisoquinoline-4-carbonitrile

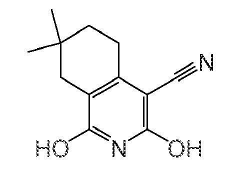

To a solution of Preparation 1 (139.50 g, 0.70mol) in methanol (436 mL) was added 2-cyanoacetamide (59.16 g, 0.70mol). Then a solution of KOH (48.56 g, 0.87mol) in MeOH (315mL) was added dropwise and the mixture stirred for an hour at room temperature and at reflux for 3h. Then the reaction mixture was allowed to cool down to room temperature and concentrated HCl was added until pH 3-4. The solid obtained was filtered and washed with methanol and diethyl ether. The solid was recrystallized in acetic acid (1000mL), filtered and washed with acetic acid and diethyl ether. After drying the solid in the vacuum oven at 120°C the desired compound (144.32g, 89% yield) was obtained as a yellow solid.
LRMS: m/z 219 (M+1)⁺
Retention time: 10.22 min (Method C)

### Preparation 3

### 1,3-dichloro-7,7-dimethyl-5,6,7,8-tetrahydroisoquinoline-4-carbonitrile

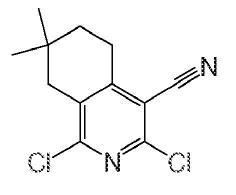

In a high pressure reactor containing Preparation 2 (14.2g, 0.07mol) was added POCl₃ 860mL, 0.66mol) and the mixture stirred at 170°C overnight. The reaction mixture was then concentrated and the residue redissolved in dichloromethane and poured over ice/water. The mixture was neutralized with concentrated NaOH and the organic layer separated, washed with 4% solution of NaHCO₃ and brine, dried over sodium sulphate and concentrated. A light brown solid (10.23g) was obtained as the desired compound (yield=62%).
LRMS: no signal
Retention time: 7.15 min (Method B)

### Preparation 4

### 3-chloro-1-ethyl-7,7-dimethyl-5,6,7,8-tetrahydroisoquinoline-4-carbonitrile

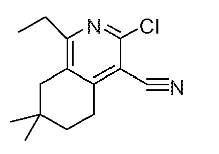

To a mixture of Preparation 3 (3.28g, 12.86mmol), ethylboronic acid (1g, 13.53mmol) and potassium carbonate (5.33g, 38.56mmol) in dioxane (82mL), was added Pd(PPh₃)₄ (0.15g, 0.13mmol) and the mixture stirred at 110°C under nitrogen atmosphere overnight. Ethyl acetate was added and the organic layer washed with water twice and brine, dried over magnesium sulphate and concentrated. The desired compound was obtained as oil (88% yield).
LRMS: no signal
Retention time: 16.47 min (Method C)

### Preparation 5

### 1-ethyl-7,7-dimethyl-5,6,7,8-tetrahydroisoquinoline-4-carbonitrile

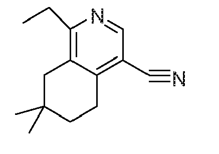

To a mixture of Preparation 4 (3.86g, 11.39mmol), triethylamine (1.97mL, 14.13mmol) in methanol (100mL) was added 20% palladium hydroxide on carbon (0.45g, 0.64mmol). The reaction mixture was hydrogenated at 6psi for 45min. The catalyst was filtered off and the filtrate concentrated. The residue was redissolved in ethyl acetate and washed with water. The organic layer was dried over magnesium sulphate and concentrated to yield a white solid (50% yield) as the desired compound.
LRMS: m/z 215 (M+1)⁺
Retention time: 5.93 min (Method B)

### Preparation 6

### 1-ethyl-7,7-dimethyl-5,6,7,8-tetrahydroisoquinoline-4-carboxylic acid

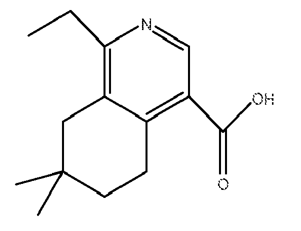

Preparation 5 (1.45g, 6.77mmol) was dissolved in ethanol (40mL) and concentrated NaOH (12.7mL, 101.6mmol) was added. The mixture was stirred overnight at 110°C. The solvent was removed under vacuum and the residue redissolved in water and acidified with acetic acid until pH 6. This aqueous layer was extracted with ethyl acetate twice. The combined organic layer was washed with water and brine, dried over magnesium sulphate and concentrated. A yellow solid was obtained (33% yield) as a desired compound.
LRMS: m/z 234 (M+1)⁺
Retention time: 4.13 min (Method B)

### Preparation 7

### 3-chloro-1-cyclopropyl-7,7-dimethyl-5,6,7,8-tetrahydroisoquinoline-4-carbonitrile

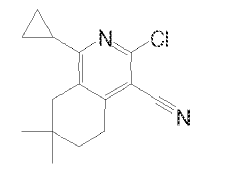

Obtained (52%) from Preparation 3 and cyclopropylboronic acid following the experimental procedure describe for Preparation 4.
LRMS: no signal
Retention time: 7.66 min (Method B)

### Preparation 8

### 1-cyclopropyl-7,7-dimethyl-5,6,7,8-tetrahydroisoquinoline-4-carbonitrile

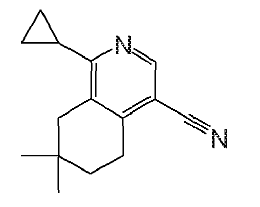

Obtained (70%) from Preparation 7 following the experimental procedure describe for Preparation 5.
LRMS: m/z 227 (M+1)⁺
Retention time: 6.98min (Method B)

### Preparation 9

### 1-cyclopropyl-7,7-dimethyl-5,6,7,8-tetrahydroisoquinoline-4-carboxylic acid

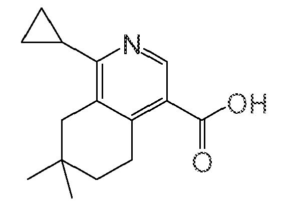

Obtained (30%) from Preparation 8 following the experimental procedure describe for Preparation 6.
LRMS: m/z 364 (M+1)⁺
Retention time: 19.07 min (Method B)

### Preparation 10

### (Z)-N'-hydroxy-4-(hydroxymethyl)benzimidamide

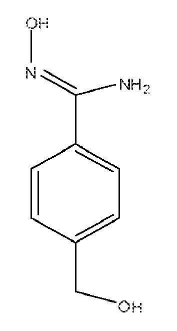

Obtained from 4-(hydroxymethyl)benzonitrile (100% yield) following the general method 1.
LRMS: m/z 167 (M+1)⁺
Retention time: 0.68 min (Method B)

### Preparation 11

### (E)-N'-hydroxy-4-sulfamoylbenzimidamide

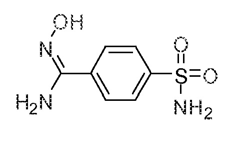

Obtained (100% yield) from 4-cyanobenzenesulfonamide following the general method 1.
LRMS: m/z 216 (M+1)⁺
Retention time: 0.70 min (Method B)
1H NMR (200 MHz, DMSO-D₆) δ ppm 5.9 (s, 2 H) 7.8 (s, 4 H).

### Preparation 12

### Ethyl 3-(4-(4-cyanophenyl)piperazin-1-yl)propanoate

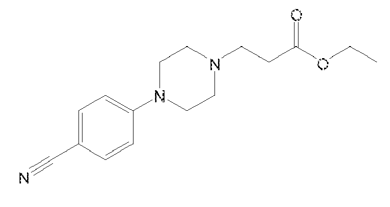

To a solution of 4-(piperazin-1-yl)benzonitrile (3g, 16.02mmol) in anhydrous acetonitrile (40mL) under nitrogen atmosphere was added Yb(OTf)₃ (0.2g, 0.32mmol) and then ethyl acrylate (3.49mL, 32.04mmol). The reaction mixture was stirred overnight at room temperature. The catalyst was filtered off and the filtrate concentrated and chromatographied to provide the title compound (86% yield) of a solid.
LRMS: m/z 288 (M+1)⁺
Retention time: 3.60 min (Method B)
1H NMR (200 MHz, CHLOROFORM-D) δ ppm 1.3 (t, *J*=7.2 Hz, 3 H) 2.6 (m, 6 H) 2.8 (t, *J*=7.0 Hz, 2 H) 3.3 (m, 4 H) 4.2 (q, *J*=7.0 Hz, 2 H) 6.8 (d, *J*=8.6 Hz, 2 H) 7.5 (d, *J*=8.6 Hz, 2 H)

### Preparation 13

### (Z)-ethyl 3-(4-(4-(N'-hydroxycarbamimidoyl)phenyl)piperazin-1-yl)propanoate

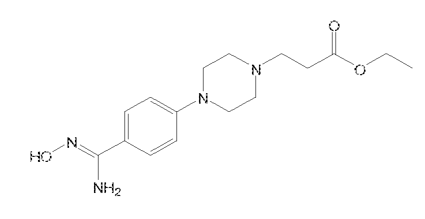

Obtained (37% yield) from Preparation 12 following the general method 1.
LRMS: m/z 321 (M+1)⁺
Retention time: 0.58 min (Method B)
1H NMR (200 MHz, DMSO-D6) δ ppm 1.2 (t, *J*=7.2 Hz, 3 H) 2.5 (m, 8 H) 2.6 (t, *J*=6.4 Hz, 2 H) 3.1 (m, 2 H) 4.1 (q, *J*=7.3 Hz, 2 H) 5.6 (s, 2 H) 6.9 (d, *J*=9.0 Hz, 2 H) 7.5 (d, *J*=9.0 Hz, 2 H) 8.3 (s, 1H).

### Preparation 14

### Ethyl 3-(4-(4-(5-(1-ethyl-7,7-dimethyl-5,6,7,8-tetrahydroisoquinolin-4-yl)-1,2,4-oxadiazol-3-yl)phenyl)piperazin-1-yl)propanoate

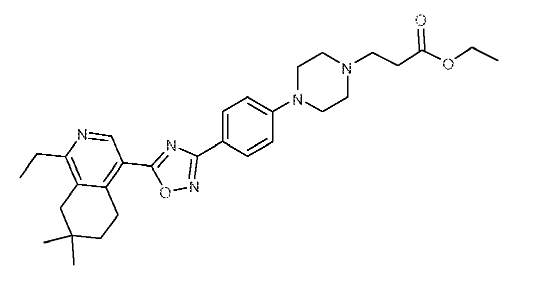

Obtained (8% yield) from Preparation 13 and Preparation 6 following the general method 2.
LRMS: m/z 518 (M+1)⁺
Retention time: 6.63 min (Method B)

### Preparation 15

### Methyl 3-(4-cyanophenylsulfonamido)propanoate

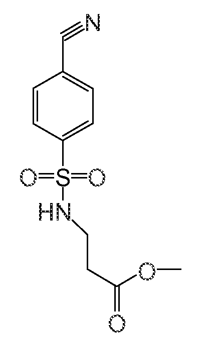

To a solution of 4-cyanobenzenesulfonamide (2g, 11mmol) in ACN (8mL) was added DBU (0.8mL, 5.48mmol) and methyl acrylate (1.42g, 16.49mmol) and the mixture stirred at room temperature for 2h. The crude was purified according to the general purification method to give 0.6g (41% yield) of the desired compound as a white solid.
LRMS: m/z 267 (M-1)⁻
Retention time: 4.74 min (Method B)

### Preparation 16

### (E)-Methyl 3-(4-(N'-hydroxycarbamimidoyl)phenylsulfonamido)propanoate

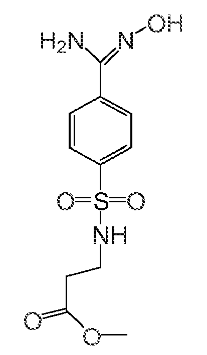

Obtained (100% yield) from Preparation 15 following the general method 1.
1H NMR (200 MHz, DMSO-D₆) δ ppm 2.0 (t, *J*=7.0 Hz, 2 H) 2.5 (s, 3 H) 2.9 (t, *J*=7.0 Hz, 2 H) 5.9 (s, 2 H) 7.8 (m, 4 H)

### Preparation 17

### Methyl 3-(4-(5-(1-ethyl-7,7-dimethyl-5,6,7,8-tetrahydroisoquinolin-4-yl)-1,2,4-oxadiazol-3-yl)phenylsulfonamido)propanoate

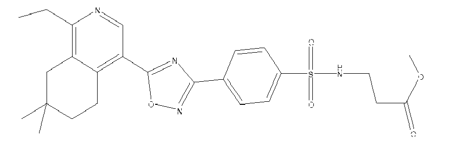

Obtained (20 % yield) from Preparation 16 and Preparation 6 following the general method 2.
LRMS: no signal
Retention time: 7.21 min (Method B)

### Preparation 18

### 4-cyano-2,6-dimethylphenyl trifluoromethanesulfonate

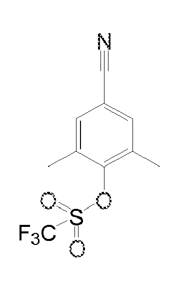

To a suspension of 4-hydroxy-3,5-dimethylbenzonitrile (5.10g, 34.65mmol) in DCM (100mL) was added triethylamine (7.25mL, 52.02mmol). To the solution obtained was added 1,1,1-trifluoro-N-phenyl-N-(trifluoromethylsulfonyl)methansulfonamide (14.9g, 41.7mmol) and the mixture stirred overnight at room temperature. More DCM was added and then washed with 0.5N NaOH, water and brine. The organic layer was dried over magnesium sulphate and concentrated to yield 11.8g of the desired compound as a solid (yield=100%).
LRMS: no signal
Retention time: 6.90 min (Method B)

### Preparation 19

### (E)-Methyl 3-(4-cyano-2,6-dimethylphenyl)acrylate

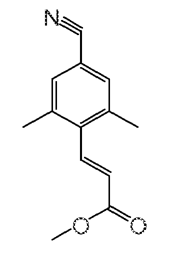

To a mixture of Preparation 18 (6.07g, 21.74mmol), methyl acrylate (5.87mL, 65.18mmol), 1,3-bis-(diphenylphosphino)propane (0.45g, 1.09mmol) and triethylamine (6.06mL, 43.48mmol) in DMF (30mL), was added palladium acetate (0.25g, 1.11mmol) under nitrogen atmosphere. The reaction mixture was stirred overnight at 110°C. After cooling to room temperature, the mixture was poured over water and extracted with diethyl ether twice. The combined organic layer was washed with water and brine, dried over magnesium sulphate and concentrated. A brown oil was obtained (68% yield) as the desired compound.
LRMS: no signal
Retention time: 6.13 min (Method B)

### Preparation 20

### (E)-Methyl 3-(4-((Z)-N'-hydroxycarbamimidoyl)-2,6-dimethylphenyl)acrylate

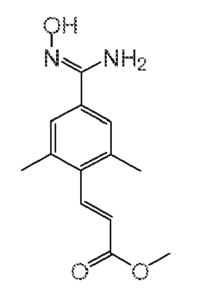

Obtained (75% yield) from Preparation 19 following the general method 1.
LRMS: m/z 249 (M+1)⁺
Retention time: 3.88 min (Method B)

### Preparation 21

### (E)-Methyl 3-(4-(N'-hydroxycarbamimidoyl)-2,6-dimethylphenyl)propanoate

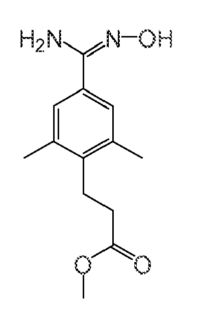

Preparation 20 (1.1 g, 4.43mmol) was dissolved in methanol (35mL) and sodium acetate (0.55g, 6.70mmol) was added. Finally palladium chloride (0.16g, 0.90mmol) was added and the mixture hydrogenated at 15 psi for 5h. The catalyst was filtered off and the filtrate concentrated. The residue was redissolved in DCM and washed with water. The organic layer was dried over magnesium sulphate and concentrated to yield an oil (82% yield) as the desired compound.
LRMS: m/z 251 (M+1)⁺
Retention time: 3.64 min (Method B)

### Preparation 22

### Methyl 3-(4-(5-(1-ethyl-7,7-dimethyl-5,6,7,8-tetrahydroisoquinolin-4-yl)-1,2,4-oxadiazol-3-yl)-2,6-dimethylphenyl)propanoate

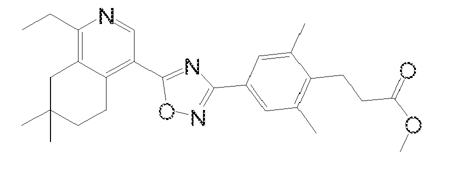

Obtained (56% yield) from Preparation 21 and Preparation 6 following the general method 2.
LRMS: m/z 448 (M+1)⁺
Retention time: 8.07 min (Method B)

### Preparation 23

### (E)-tert-butyl 3-(4-cyano-2,6-dimethylphenyl)acrylate

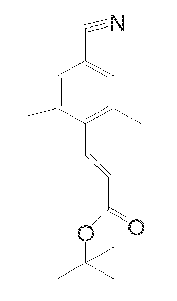

Obtained (64%) from Preparation 18 and *tert*-butyl acrylate following the experimental procedure describe for Preparation 19.
LRMS: m/z 258 (M+1)⁺
Retention time: 7.18 min (Method B)

### Preparation 24

### (E)-tert-butyl 3-(4-((E)-N'-hydroxycarbamimidoyl)-2,6-dimethylphenyl)acrylate

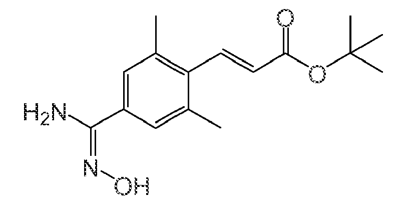

Obtained (76%) from Preparation 23 following the general method 1.
LRMS: m/z 291 (M+1)⁺
Retention time: 4.89 min (Method B)

### Preparation 25

### (E)-tert-butyl 3-(4-(N'-hydroxycarbamimidoyl)-2,6-dimethylphenyl)propanoate

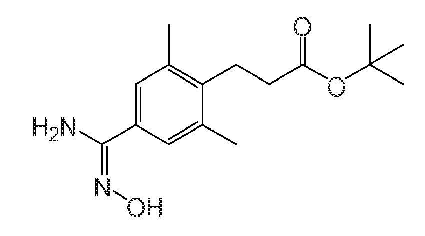

Obtained (77%) from Preparation 24 following the experimental procedure describe for Preparation 21.
LRMS: m/z 293 (M+1)⁺
Retention time: 4.66 min (Method B)

### Preparation 26

### Tert-butyl 3-(4-(5-(1-cyclopropyl-7,7-dimethyl-5,6,7,8-tetrahydroisoquinolin-4-yl)-1,2,4-oxadiazol-3-yl)-2,6-dimethylphenyl)propanoate

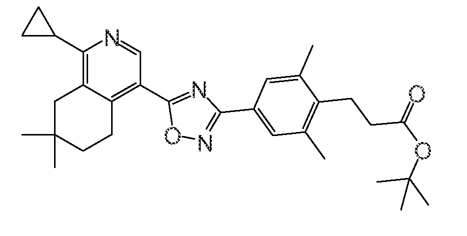

Obtained (30% yield) from Preparation 25 and Preparation 9 following the general method 2.
LRMS: m/z 502 (M+1)⁺
Retention time: 7.18 min (Method B)

### Preparation 27

### 7,7-dimethyl-5,6,7,8-tetrahydroisoquinoline-4-carbonitrile

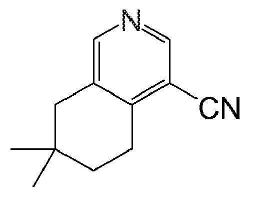

Obtained (100%) from Preparation 3 following the experimental procedure describe for Preparation 5.
LRMS: m/z 187 (M+1)⁺
Retention time: 5.96 min (Method B)

### Preparation 28

### 7,7-dimethyl-5,6,7,8-tetrahydroisoquinoline-4-carboxylic acid

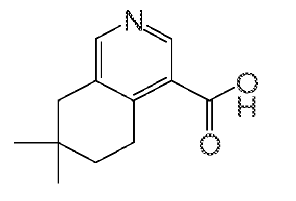

Obtained (75%) from Preparation 27 following the experimental procedure describe for Preparation 6.
LRMS: m/z 206 (M+1)⁺
Retention time: 3.75 min (Method B)

### Preparation 29

### Tert-butyl 3-(4-(5-(7,7-dimethyl-5,6,7,8-tetrahydroisoquinolin-4-yl)-1,2,4-oxadiazol-3-yl)-2,6-dimethylphenyl)propanoate

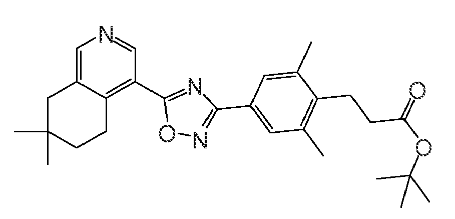

Obtained (42% yield) from Preparation 28 and Preparation 25 following the general method 2.
LRMS: m/z 462 (M+1)⁺
Retention time: 6.63min (Method B)

### Preparation 30

### Ethyl 3-(4-(5-(7,7-dimethyl-5,6,7,8-tetrahydroisoquinolin-4-yl)-1,2,4-oxadiazol-3-yl)-3-methylphenyl)propanoate

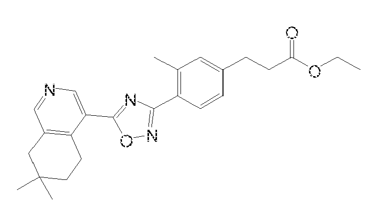

Obtained (29% yield) from Preparation 28 and Preparation 31 following the general method 2.
LRMS: m/z 420 (M+1)⁺
Retention time: 7.92min (Method B)

### Preparation 31

### (Z)-Ethyl 3-(4-(N'-hydroxycarbamimidoyl)-3-methylphenyl)propanoate

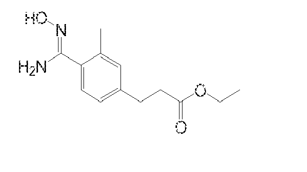

Obtained (56% yield) from Preparation 32 following the general method 1.
LRMS: m/z 251 (M+1)⁺
Retention time: 3.56 min (Method B)

### Preparation 32

### Ethyl 3-(4-cyano-3-methylphenyl)propanoate

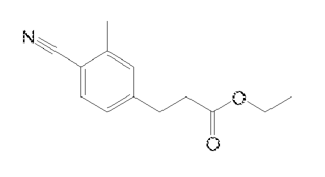

Preparation 33 (7.7g, 33.63mmol) was dissolved in methanol (200mL) and under nitrogen atmosphere was added Pd/C (0.07g, 0.07mmol). The mixture was hydrogenated at 1 psi for 1 h. The catalyst was filtered off and the filtrate concentrated to give the title compound (yield=97%).
LRMS: m/z 218 (M+1)⁺
Retention time: 6.06 min (Method B)
1H NMR (200 MHz, CHLOROFORM-d) δ ppm 1.2 (t, J=7.2Hz, 3 H) 2.5 (s, 3 H) 2.6 (t, J=7.6Hz, 2H) 2.9 (t, J=7.6Hz, 2H) 4.1 (q, J=7.2Hz, 2H) 7.1 (m, 2H) 7.5 (d, J=7.8Hz, 1H)

### Preparation 33

### (E)-Ethyl 3-(4-cyano-3-methylphenyl)acrylate

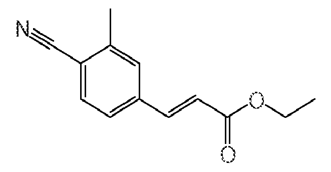

Obtained (66% yield) from 4-bromo-2-methylbenzonitrile and ethyl acrylate following the experimental procedure described for Preparation 19.
LRMS: m/z 216 (M+1)⁺
Retention time: 6.29 min (Method B)
1H NMR (200 MHz, CHLOROFORM-*d*) δ ppm 1.3 (t, J=7.2Hz, 3 H) 2.6 (s, 3 H) 4.3 (q, J=7.2Hz, 2H) 6.5 (d, J=16Hz, 1H) 7.5 (m, 4H)

### Preparation 34

### Tert-butyl 2-(3-(4-(5-(7,7-dimethyl-5,6,7,8-tetrahydroisoquinolin-4-yl)-1,2,4-oxadiazol-3-yl)-2,6-dimethylphenyl)propanamido)acetate

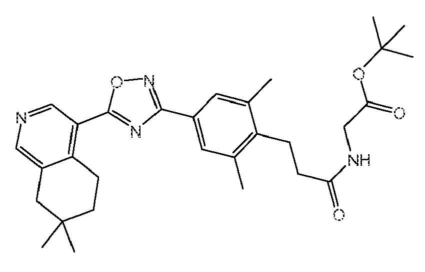

A mixture of Example 9 (100mg, 0.25mmol), tert-butyl 2-aminoacetate hydrochloride (62mg, 0.37mmol), HATU (94mg, 0.25mmol) and DIEA (130 µl, 0.75mmol) in DMF (3mL) was stirred overnight at r.t. The mixture was poured over ice/water and stirred for 15min. The solid formed was filtered off, washed with water and dried in the vacuum oven and used without further purification.
LRMS: m/z 519 (M+H)⁺
Retention time: 7.72min (Method B)

### Preparation 35

### Methyl 3-(4-(5-(7,7-dimethyl-5,6,7,8-tetrahydroisoquinolin-4-yl)-1,2,4-oxadiazol-3-yl)phenyl)propanoate

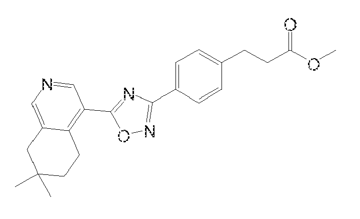

Obtained (58%) from Preparation 37 and Preparation 28 following the general method 2.
LRMS: m/z 392 (M+H)⁺
Retention time: 7.63min (Method B)

### Preparation 36

### (Z)-Methyl 3-(4-(N'-hydroxycarbamimidoyl)phenyl)propanoate

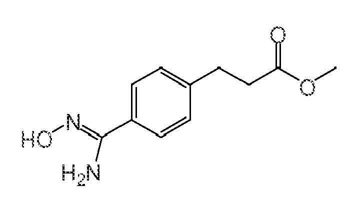

Obtained (69% yield) from Preparation 38 following the general method 1.
LRMS: m/z 223 (M+H)⁺
Retention time: 3.07min (Method B)

### Preparation 37

### Methyl 3-(4-cyanophenyl)propanoate

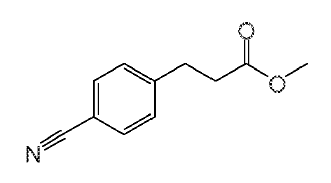

To a solultion of Preparation 39 (680mg, 3.88mmol) in methanol (8mL) and dichloromethane (8mL) at 0°C was added trimethylsilyldiazomethane (2M in hexane, 3.9mL) until a yellow color persisted. The reaction mixture was stirred at room temperature for 1h and was concentrated to dryness to give the title compound, which was used without further purification (87% yield).
LRMS: no signal
Retention time: 5.42min (Method B)
1H NMR (200 MHz, CHLOROFORM-D) δ ppm 2.7 (t, *J*=7.4 Hz, 2 H) 3.0 (t, *J*=7.4 Hz, 2 H) 3.7 (s, 3 H) 7.3 (d, *J*=8.4 Hz, 2 H) 7.6 (d, *J*=8.4 Hz, 2 H)

### Preparation 38

### 3-(4-cyanophenyl)propanoic acid

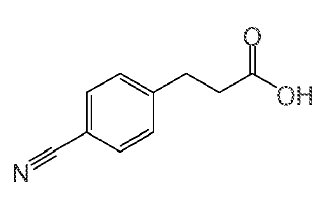

Obtained (52% yield) from Preparation 40 following the experimental procedure described for Preparation 21.
LRMS: m/z 174(M-H)⁻
Retention time: 4.70(Method B)

### Preparation 39

### (E)-3-(4-cyanophenyl)acrylic acid

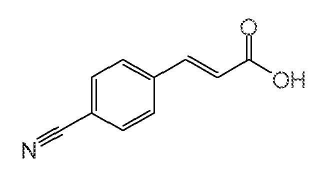

A suspension of 4-formylbenzonitrile (1g, 7.63mmol) and malonic acid (1.59g, 15.28mmol) in pyridine (10mL) was stirred at 45°C until a solution was obtained. Then piperidine (0.15mL, 1.53mmol) was added and the reaction mixture stirred at 85°C for 30min and at reflux for 4h.5N HCl was added dropwise and the white solid formed was filtered off, washed with water and dried in the vacuum oven (93% yield).
LRMS: m/z 172 (M-H)⁻
Retention time: 4.84min (Method B)

### Preparation 40

### Methyl 4-(4-(5-(7,7-dimethyl-5,6,7,8-tetrahydroisoquinolin-4-yl)-1,2,4-oxadiazol-3-yl)phenyl)butanoate

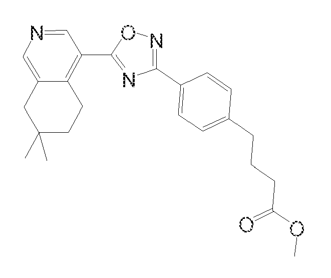

Obtained (51% yield) from Preparation 42 and Preparation 28 following the general method 2.
LRMS: m/z 406 (M+H)⁺
Retention time: 7.78min (Method B)

### Preparation 41

### (Z)-Methyl 4-(4-(N'-hydroxycarbamimidoyl)phenyl)butanoate

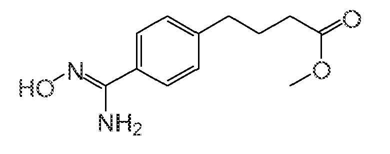

Obtained (100% yield) from Preparation 43 following the general method 1.
LRMS: m/z 237 (M+H)⁺
Retention time: 3.39min (Method B)

### Preparation 42

### Methyl 4-(4-cyanophenyl)butanoate

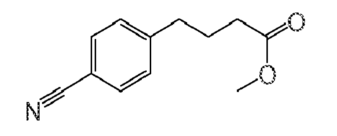

Obtained (99% yield) from Preparation 44 following the experimental procedure described for Preparation 38.
LRMS: no signal
Retention time: 5.70min (Method B)

### Preparation 43

### 4-(4-cyanophenyl)butanoic acid

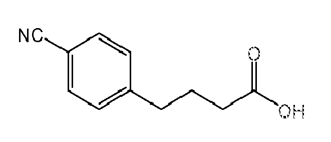

4(4-iodophenyl)butanoic acid (0.5 g, 1.72mmol) was dissolved in DMF (5mL) and dicyanozinc (0.25g, 2.09mmol) and Pd(PPh₃)₄ (0.22g, 0.19mmol) were added. The mixture was heated under nitrogen atmosphere at 120°C for 2h. The mixture was filtered through Decalite and washed with ethyl acetate and water. 2N NaOH was added and the organic layer separated. The aqueous phase was acidified with 5N HCl and extracted twice with ethyl acetate. This organic layer was washed with water, brine, dried over magnesium sulphate and concentrated to give an oil as the title compound. Yield=83%.
LRMS: m/z 188 (M-H)⁻
Retention time: 5.08min (Method B)

### Preparation 44

### Tert-butyl 6-(5-(7,7-dimethyl-5,6,7,8-tetrahydroisoquinolin-4-yl)-1,2,4-oxadiazol-3-yl)-1,2,3,4-tetrahydronaphthalen-2-ylcarbamate

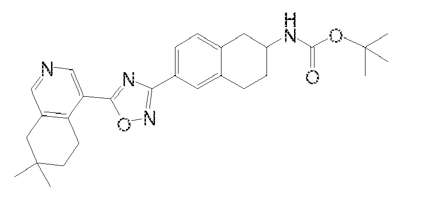

Obtained (55% yield) from Preparation 46 and Preparation 28 following the general method 2.
LRMS: m/z 475 (M+H)⁺
Retention time: 7.93min (Method B)

### Preparation 45

### (Z)-tert-butyl 6-(N'-hydroxycarbamimidoyl)-1,2,3,4-tetrahydronaphthalen-2-ylcarbamate

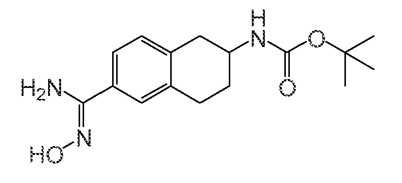

Obtained (100% yield) from Preparation 47 following the general method 1.
LRMS: m/z 306 (M+1)⁺
Retention time: 4.09min (Method B)

### Preparation 46

### Tert-butyl 6-cyano-1,2,3,4-tetrahydronaphthalen-2-ylcarbamate

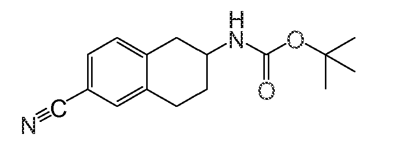

Obtained (79% yield) from Preparation 48 following the experimental procedure described for Preparation 44.
LRMS: m/z 273 (M+1)⁺
Retention time: 6.31 min (Method B)

### Preparation 47

### Tert-butyl 6-bromo-1,2,3,4-tetrahydronaphthalen-2-ylcarbamate

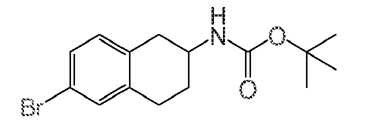

A mixture of Preparation 49 (1.31 g, 5.79mmol), BOC₂O (1.39g, 6.37mmol) and Et₃N (1.21 mL, 8.68mmol) in DCM (100mL) was stirred at r.t. overnight. The reaction was diluted with DCM, washed with saturated aqueous NaHCO₃, dried over magnesium sulphate and concentrated in vacuo to afford the crude material, 1.91 g of a grey solid (99% yield).
LRMS: m/z 326, 328 (M+1)⁺
Retention time: 7.14min (Method B)

### Preparation 48

### 6-bromo-1,2,3,4-tetrahydronaphthalen-2-amine

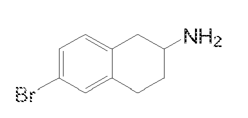

To a solution of the 6-bromo 2-tetralone (2g, 8.89mmol) and NH₄OAc (5.52g, 71.61mmol) in MeOH (100 mL) was added NaCNBH₃ (0.67g, 10.66mmol) at room temperature. The resulting yellow solution was stirred at that temperature for 20 hours. The reaction mixture was acidified with 2 M HCl, stirred for 10min and methanol evaporated. The mixture was extracted with CH₂Cl₂ twice. The aqueous layer was basified with 1.0 N NaOH to pH 10 then extracted with CH₂Cl₂ two times. The extracts are dried over anhydrous MgSO₄ and concentrated in vacuo to afford 1.31 g (65percent yield) of the desired product as a yellow oil which is used without further purification.
LRMS: m/z 226.1 (M+H)⁺, 209 (M+H- NH3)⁺.
Retention time: 3.84min (Method B)
¹H NMR (300 MHz, CD₃OD) δ 7.27-7.35 (m, 8H), 7.05 (d, J = 8.4 Hz, 4H), 3.56 (m, IH), 3.17 (dd, J = 3.9, 16.2 Hz, IH), 2.95 (m, 2H), 2.81 (dd, J = 9.9, 16.2 Hz, IH), 2.19-2.29 (m, IH), 1.79-1.92 (m, IH)

### Preparation 49

### Ethyl 2-(6-(5-(7,7-dimethyl-5,6,7,8-tetrahydroisoquinolin-4-yl)-1,2,4-oxadiazol-3-yl)-1,2,3,4-tetrahydronaphthalen-2-ylamino)acetate

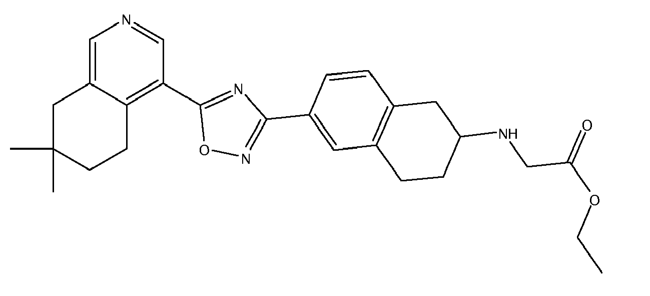

A mixture of Example 18 (172mg, 0.46mmol), ethyl 2-oxoacetate (146 I, 0.74mmol) and AcOH (239µl, 4.18mmol) in methanol (5mL) was stirred at room temperature for 1 h. Then NaBH₃CN (20mg, 0.32mmol) was added and the reaction mixture stirred at r.t. overnight. Solvent was concentrated and the residue dissolved in ethyl acetate, washed with water and brine, dried over magnesium sulphate, filtered and concentrated. The crude obtained was purified according the General purification method to give the desired compound (50% yield).
LRMS: m/z 461 (M+H)⁺
Retention time: 5.74min (Method B)

### Preparation 50

### 3-(4-allyl-3,5-dimethylphenyl)-5-(7,7-dimethyl-5,6,7,8-tetrahydroisoquinolin-4-yl)-1,2,4-oxadiazole

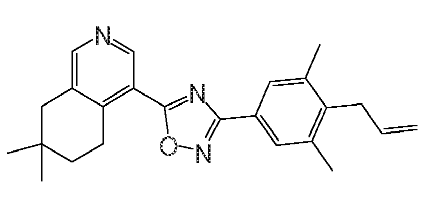

Obtained (55% yield) from Preparation 28 and Preparation 52 following the general method 2.
LRMS: m/z 374 (M+H)⁺
Retention time: 8.46min (Method B)

### Preparation 51

### (Z)-4-allyl-N'-hydroxy-3,5-dimethylbenzimidamide

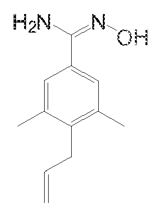

Obtained (69% yield) from Preparation 53 following the general method 1.
LRMS: m/z 205 (M+H)⁺
Retention time: 4.07min (Method B)

### Preparation 52

### 4-allyl-3,5-dimethylbenzonitrile

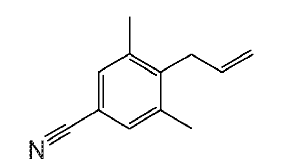

To a solution of Preparation 18 (5g, 14.74mmol) in DMF (175mL) under nitrogen atmosphere, was added allyltributylstannane (5.50mL, 17.74mmol) and Pd(PPh3)4 (1.71 g, 1.48mmol) and the mixture stirred overnight at 90°C. The reaction mixture was poured over ice/water and ethyl acetate was added. The mixture was filtered over Decalite and the organic layer separated, washed with water and brine, dried over magnesium sulphate and concentrated. The crude obtained was used without further purification (80% yield).
LRMS: no signal
Retention time: 6.69min (Method B)

### Preparation 53

### 2-(4-(5-(7,7-dimethyl-5,6,7,8-tetrahydroisoquinolin-4-yl)-1,2,4-oxadiazol-3-yl)-2,6-dimethylphenyl)acetaldehyde

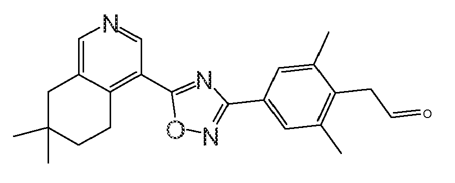

To a solution of example 22 (660mg, 1.62mmol) in methanol (18mL) and water (2mL) was added NalO₄ (520mg, 2.05mmol) and the mixture stirred overnight at room temperature. Methanol was concentrated and the residue dissolved in ethyl acetate and water. Organic layer was separated, washed with water and brine, dried over magnesium sulphate and concentrated to give the title compound (87% yield).
LRMS: m/z 376 (M+H)⁺
Retention time: 6.12min (Method B)

### Preparation 54

### methyl 3-(5-(5-(7,7-dimethyl-5,6,7,8-tetrahydroisoquinolin-4-yl)-1,2,4-oxadiazol-3-yl)-3-ethyl-6-methylpyridin-2-yl)propanoate

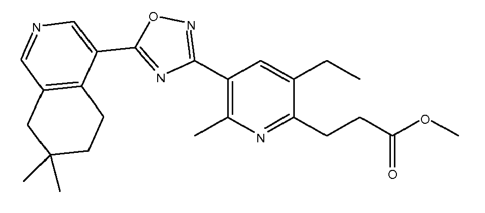

Obtained (60% yield) from Preparation 28 and Preparation 56 following the general method 2.
LRMS: m/z 435 (M+1)⁺
Retention time: 7.72 min (Method B)

### Preparation 55

### (Z)-methyl 3-(3-ethyl-5-(N'-hydroxycarbamimidoyl)-6-methylpyridin-2-yl)propanoate

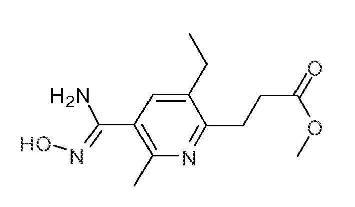

Obtained (51% yield) from Preparation 57 and Preparation following the general method 1.
LRMS: m/z 266 (M+1)⁺
Retention time: 3.00 min (Method B)

### Preparation 56

### methyl 3-(5-cyano-3-ethyl-6-methylpyridin-2-yl)propanoate

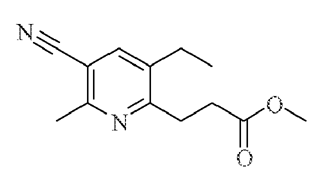

To a solution of Preparation 58 (100mg, 0,43mmol) in MeOH (4mL) was added Pd/C in catalytic quantity and submitted under hydrogen atmosphere at atmospheric pressure for 30min. After filtration of the catalyst and concentration 94 mg of the desired compound were obtained as a solid (86% yield).
LRMS: m/z 233 (M+1)⁺
Retention time: 5.73 min (Method B)

### Preparation 57

### (E)-Methyl 3-(5-cyano-3-ethyl-6-methylpyridin-2-yl)acrylate

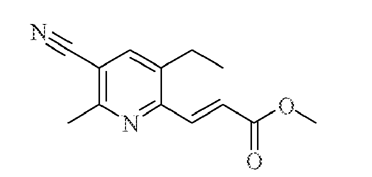

Obtained (44% yield) from Preparation 59 and methyl acrylate following the experimental procedure described for Preparation 19.
LRMS: m/z 231 (M+1)⁺
Retention time: 6.20 min (Method B)

### Preparation 58

### 6-bromo-5-ethyl-2-methylnicotinonitrile

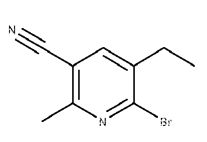

A mixture of 5-ethyl-6-hydroxy-2-methylnicotinonitrile (1,1g, 6,8mmol), phosphoryl tribromide (2g, 7mmol) and tribromophosphine (0,7mL, 7,4mmol) was stirred at 120°C for 3h. The resulting mixture was slowly added to a mixture of ice and water. DCM was added and then washed with water and brine. The organic layer was dried over magnesium sulphate and concentrated to yield 1,53g of the desired compound as a solid (94% yield).
LRMS: m/z 225, 227 (M+1)⁺
Retention time: 3.08 min (Method A)

### Preparation 59

### Tert-butyl 3-(2-chloro-4-(5-(7,7-dimethyl-5,6,7,8-tetrahydroisoquinolin-4-yl)-1,2,4-oxadiazol-3-yl)phenylsulfonamido)propanoate

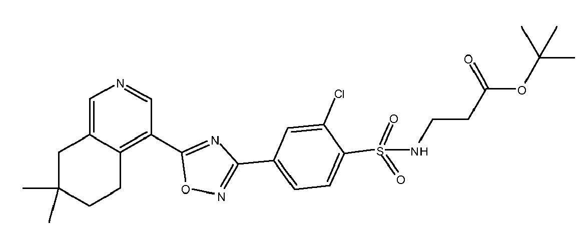

Obtained (52% yield) from Preparation 28 and Preparation 61 following the general method 2.
LRMS: m/z 547, 549 (M+1)⁺
Retention time: 7.73min (Method B)

### Preparation 60

### (Z)-Tert-butyl 3-(2-chloro-4-(N'-hydroxycarbamimidoyl)phenylsulfonamido) propanoate

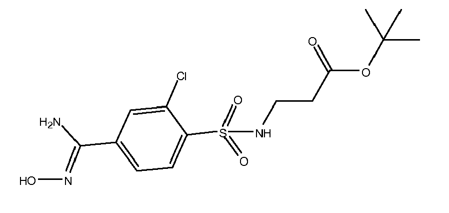

Obtained (98% yield) from Preparation 62 following the general method 1.
LRMS: m/z 378, 380 (M+1)⁺
Retention time: 5.12min (Method B)

### Preparation 61

### tert-butyl 3-(2-chloro-4-cyanophenylsulfonamido)propanoate

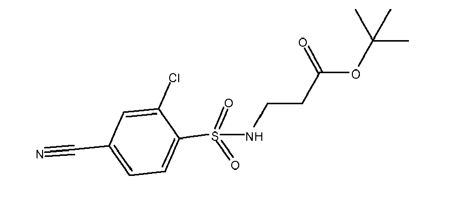

To a mixture of *tert*-butyl 3-aminopropanoate hydrochloride (1.54g, 8.48mmol) and triethylamine (2.48mL, 17.79mol) in DCM (25mL) was added 2-chloro-4-cyanobenzene-1-sulfonyl chloride (2,0g, 8.47mmol) slowly and the reaction mixture stirred for 3h at room temperature. Reaction mixture was diluted with ethyl acetate and washed with water and brine. Organic layer was dried over magnesium sulphate, filtered and concentrated to give the title compound as an oil (100% yield).
LRMS: m/z 362, 364 (M+17)⁺
Retention time: 6.10min (Method B)

### Preparation 62

### Tert-butyl 3-(4-(5-(7,7-dimethyl-5,6,7,8-tetrahydroisoquinolin-4-yl)-1,2,4-oxadiazol-3-yl)phenylsulfonamido)propanoate

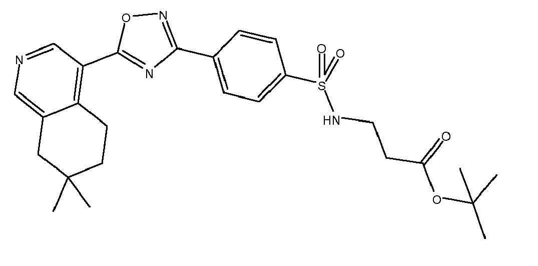

Obtained (91% yield) from Preparation 28 and Preparation 64 following the general method 2.
LRMS: m/z 513 (M+1)⁺
Retention time: 7.55min (Method B)

### Preparation 63

### (Z)-Tert-butyl 3-(4-(N'-hydroxycarbamimidoyl)phenylsulfonamido)propanoate

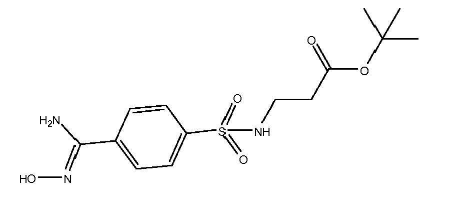

Obtained (74% yield) from Preparation 65 following the general method 1.
LRMS: m/z 344 (M+1)⁺
Retention time: 4.55min (Method B)

### Preparation 64

### Tert-butyl 3-(4-cyanophenylsulfonamido)propanoate

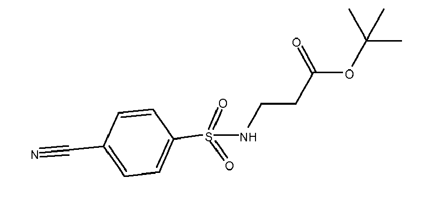

Obtained (57% yield) from 4-cyanobenzene-1-sulfonyl chloride following the experimental procedure described for Preparation 62.
LRMS: m/z 309 (M-1)⁻
Retention time: 5.88min (Method B)

### Preparation 65

### 4-(3-(4-allyl-3,5-dimethylphenyl)-1,2,4-oxadiazol-5-yl)-7,7-dimethyl-5,6,7,8-tetrahydroisoquinolin-1-amine

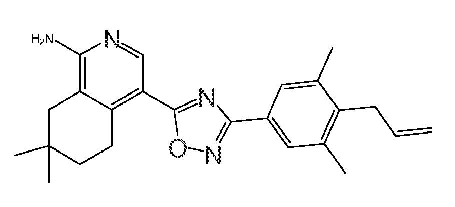

Obtained (22% yield) from Preparation 67 and Preparation 52 following the general method 2.
LRMS: m/z 389 (M+H)⁺
Retention time: 7.92min (Method B)

### Preparation 66

### 1-amino-7,7-dimethyl-5,6,7,8-tetrahydroisoquinoline-4-carboxylic acid

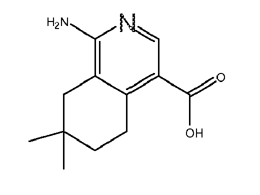

Obtained (80% yield) from Preparation 68 following the experimental procedure described for Preparation 6.
LRMS: m/z 221 (M+H)⁺
Retention time: 3.64min (Method B)

### Preparation 67

### 1-amino-7,7-dimethyl-5,6,7,8-tetrahydroisoquinoline-4-carbonitrile

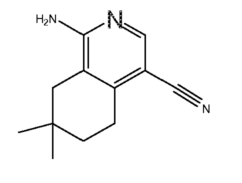

Obtained (56% yield) from Preparation 69 following the experimental procedure described for Preparation 5 and purified according the General purification method.
LRMS: m/z 202 (M+H)⁺
Retention time: 4.97min (Method B)

### Preparation 68

### 1-amino-3-chloro-7,7-dimethyl-5,6,7,8-tetrahydroisoquinoline-4-carbonitrile

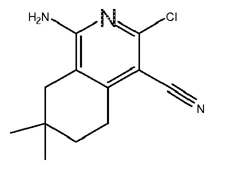

In a pressure reactor Preparation 3 (1.0g, 3.92mmol) was dissolved in 7N ammonia in methanol. The reaction mixture was stirred at 60°C for 16h. Solvent was evaporated and the residue redissolved in ethyl acetate, washed with water and brine, dried over magnesium sulphate and concentrated. The title compound was obtained as a solid (52% yield).
LRMS: m/z 236, 238 (M+H)⁺
Retention time: 6.17min (Method B)

### Preparation 69

### Methyl 4-(4-(5-(7,7-dimethyl-5,6,7,8-tetrahydroisoquinolin-4-yl)-1,2,4-oxadiazol-3-yl)-2,6-dimethylphenyl)butanoate

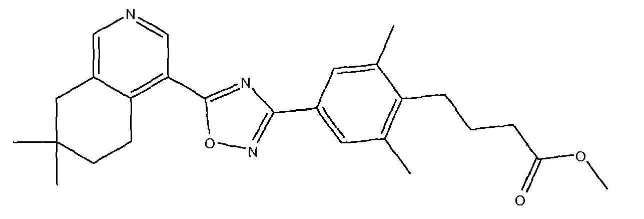

Obtained (14% yield) from Preparation 28 and Preparation 71 following the general method 2.
LRMS: m/z 434 (M+H)⁺
Retention time: 7.92min (Method B)

### Preparation 70

### (Z)-Methyl 4-(4-(N'-hydroxycarbamimidoyl)-2,6-dimethylphenyl)butanoate

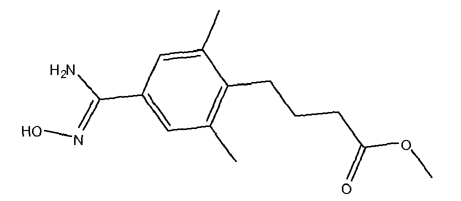

Obtained (30% yield) from Preparation 72 following the experimental procedure described for Preparation 21.
LRMS: m/z 265 (M+1)⁺
Retention time: 4.03min (Method B)

### Preparation 71

### (E)-Methyl 4-(4-((Z)-N'-hydroxycarbamimidoyl)-2,6-dimethylphenyl)but-2-enoate

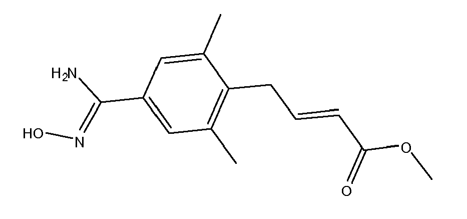

Obtained (74% yield) from Preparation 73 following the general method 1.
LRMS: m/z 263 (M+1)⁺
Retention time: 4.01 min (Method B)

### Preparation 72

### (E)-Methyl 4-(4-cyano-2,6-dimethylphenyl)but-2-enoate

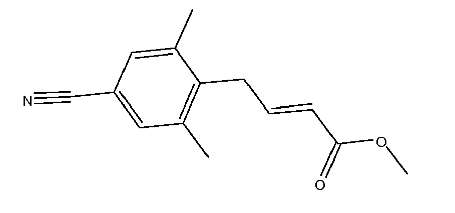

Obtained (30% yield) from Preparation 74 following the experimental procedure described for Preparation 38.
LRMS: no signal
Retention time: 6.20 min (Method B)

### Preparation 73

### (E)-4-(4-cyano-2,6-dimethylphenyl)but-2-enoic acid

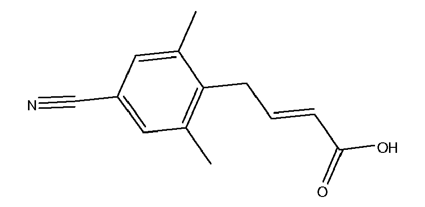

Obtained (30% yield) from Preparation 75 following the experimental procedure described for Preparation 40.
LRMS: m/z 214 (M-1)⁻
Retention time: 5.65 min (Method C)

### Preparation 74

### 3,5-dimethyl-4-(2-oxoethyl)benzonitrile

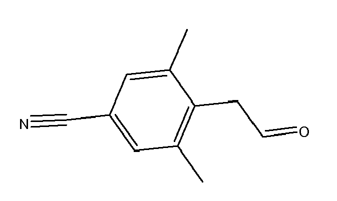

Obtained (90% yield) from Preparation 53 following the experimental procedure described for Example 22 and then following the experimental procedure described for Preparation 54.
LRMS: m/z 174 (M+1)⁺
Retention time: 5.23 min (Method C)

### Preparation 75

### 2-(4-(5-(1-amino-7,7-dimethyl-5,6,7,8-tetrahydroisoquinolin-4-yl)-1,2,4-oxadiazol-3-yl)-2,6-dimethylphenyl)acetaldehyde

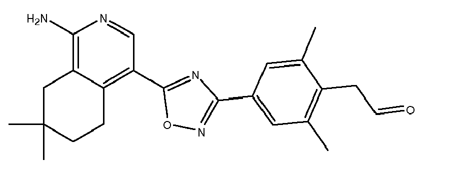

Obtained (92% yield) from Example 29 following the experimental procedure described for Preparation 54.
LRMS: m/z 391 (M+1)⁺
Retention time: 6.78 min (Method C)

### EXAMPLES

### EXAMPLE 1

### (4-(5-(1-ethyl-7,7-dimethyl-5,6,7,8-tetrahydroisoquinolin-4-yl)-1,2,4-oxadiazol-3-yl)phenyl)methanol

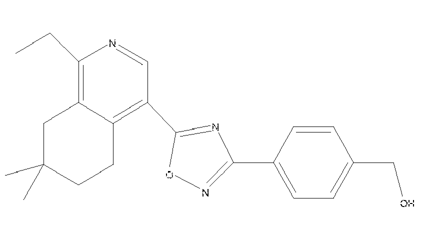

Obtained (11% yield) from Preparation 6 and Preparation 10 following the general method 2.
LRMS: m/z 364 (M+1)⁺
Retention time: 19.07 min (Method C)
1H NMR (400 MHz, CDCl₃) δ ppm 1.1 (s, 6 H) 1.3 (t, *J*=7.4 Hz, 3 H) 1.6 (m, 2 H) 2.6 (s, 2 H) 2.9 (q, *J*=7.4 Hz, 2 H) 3.3 (t, *J*=6.7 Hz, 2 H) 4.8 (d, *J*=2.3 Hz, 2 H) 7.5 (d, *J*=8.2 Hz, 2 H) 8.2 (d, *J*=8.2 Hz, 2 H) 9.1 (s, 1H)

### EXAMPLE 2

### 4-(5-(1-ethyl-7,7-dimethyl-5,6,7,8-tetrahydroisoquinolin-4-yl)-1,2,4-oxadiazol-3-yl)benzenesulfonamide

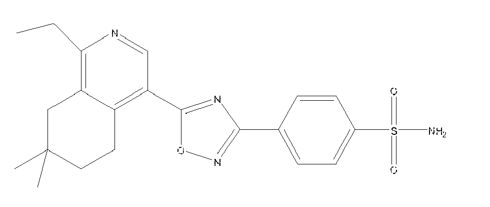

Obtained (4% yield) from Preparation 6 and Preparation 11 following the general method 2.
LRMS: m/z 364 (M+1)⁺
Retention time: 19.07 min (Method C)
1H NMR (200 MHz, DMSO-D6) δ ppm 1.0 (s, 6 H) 1.2 (t, *J*=7.2 Hz, 3 H) 1.6 (t, *J*=6.8 Hz, 2 H) 2.6 (s, 2 H) 2.8 (q, *J*=7.2 Hz, 2 H) 3.2 (m, 2 H) 8.0 (d, *J*=8.6 Hz, 2 H) 8.3 (d, *J*=8.6 Hz, 2 H) 9.0 (s, 1H).

### EXAMPLE 3

### 3-(4-(4-(5-(1-ethyl-7,7-dimethyl-5,6,7,8-tetrahydroisoquinolin-4-yl)-1,2,4-oxadiazol-3-yl)phenyl)piperazin-1-yl)propanoic acid

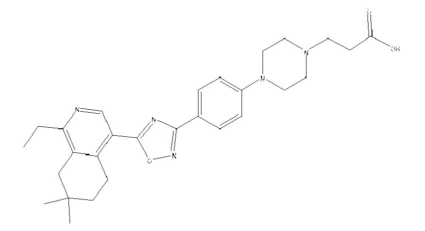

Obtained (81% yield) from Preparation 14 following the general method 3.
LRMS: m/z 490 (M+1)⁺
Retention time: 15.94 min (Method C)
¹H NMR (400 MHz, DMSO-D6) δ ppm 1.0 (m, *J*=3.9 Hz, 6 H) 1.2 (t, *J*=7.4 Hz, 3 H) 1.6 (q, 2 H) 2.4 (t, *J*=7.0 Hz, 2 H) 2.6 (m, 2 H) 2.6 (t, *J*=7.0 Hz, 2 H) 2.8 (q, *J*=7.4 Hz, 2 H) 3.2 (m, *J*=6.5, 6.5 Hz, 2 H) 3.3 (m, 8 H) 7.1 (d, *J*=8.6 Hz, 2 H) 7.9 (d, *J*=8.6 Hz, 2 H) 8.9 (s, 1H)

### EXAMPLE 4

### 3-(4-(5-(1-ethyl-7,7-dimethyl-5,6,7,8-tetrahydroisoquinolin-4-yl)-1,2,4-oxadiazol-3-yl)phenylsulfonamido)propanoic acid

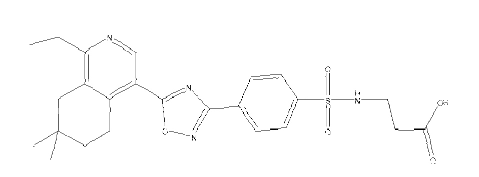

Obtained (5%) from Preparation 17 following the general method 3 and after the General purification method.
LRMS: m/z 485 (M+1)⁺
Retention time: 18.67 min (Method C)
1H NMR (200 MHz, CDCl₃) δ ppm 0.9 (m, 2 H) 1.0 (s, 6 H) 1.4 (m, 3 H) 1.6 (d, *J*=7.0 Hz, 2 H) 2.3 (m, 2 H) 2.5 (s, 2 H) 2.9 (m, 2 H) 3.3 (m, 2 H) 8.0 (d, *J*=8.2 Hz, 2 H) 8.3 (d, *J*=8.2 Hz, 2 H) 9.1 (s, 1H)

### EXAMPLE 5

### 3-(4-(5-(1-ethyl-7,7-dimethyl-5,6,7,8-tetrahydroisoquinolin-4-yl)-1,2,4-oxadiazol-3-yl)-2,6-dimethylphenyl)propanoic acid

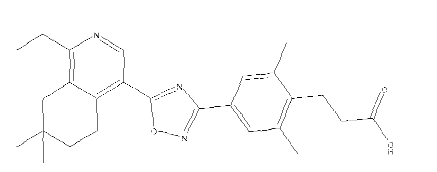

Obtained (75%) from Preparation 22 following the general method 3.
LRMS: m/z 434 (M+1)⁺
Retention time: 20.70 min (Method C)
¹H NMR (400 MHz, DMSO-D₆) δ ppm 1.0 (s, 6 H) 1.2 (t, *J*=7.4 Hz, 3 H) 1.6 (t, *J*=6.3 Hz, 2 H) 2.4 (s, 6 H) 2.6 (s, 2 H) 2.8 (q, *J*=7.4 Hz, 2 H) 2.9 (m, 2 H) 3.2 (d, *J*=5.9 Hz, 2 H) 3.3 (s, 2 H) 7.7 (s, 2 H) 9.0 (s, 1H) 12.3 (s, 1H)

### EXAMPLE 6

### 3-(4-(5-(1-ethyl-7,7-dimethyl-5,6,7,8-tetrahydroisoquinolin-4-yl)-1,2,4-oxadiazol-3-yl)-2,6-dimethylphenyl)propanamide

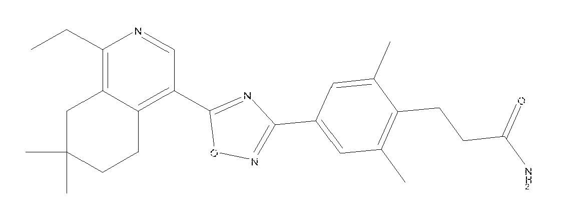

Obtained (45% yield) from Example 5 following the general method 5.
LRMS: m/z 433 (M+1)⁺
Retention time: 19.95 min (Method C)
1H NMR (200 MHz, DMSO-D6) δ ppm 1.0 (m, 6 H) 1.2 (t, *J*=7.4 Hz, 3 H) 1.6 (t, *J*=6.4 Hz, 2 H) 2.2 (dd, *J*=10.0, 6.8 Hz, 2 H) 2.4 (s, 6 H) 2.6 (s, 2 H) 2.8 (m, 4 H) 3.2 (t, *J*=6.1 Hz, 2 H) 6.9 (s, 1H) 7.4 (s, 1H) 7.7 (s, 2 H) 9.0 (s, 1H)

### EXAMPLE 7

### 2-(4-(5-(1-ethyl-7,7-dimethyl-5,6,7,8-tetrahydroisoquinolin-4-yl)-1,2,4-oxadiazol-3-yl)-2,6-dimethylphenyl)ethanamine

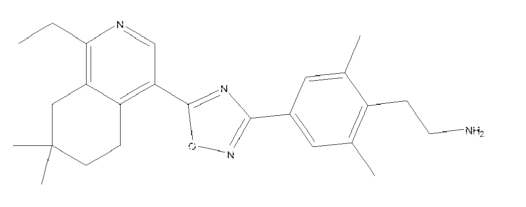

Obtained (26% yield) from Example 5 following the general method 6.
LRMS: m/z 405 (M+1)⁺
Retention time: 14.51 min (Method C)
¹H NMR (200 MHz, CDCl₃) δ ppm 1.1 (s, 6 H) 1.3 (t, *J*=7.6 Hz, 3 H) 1.6 (t, *J*=6.6 Hz, 2 H) 1.7 (s, 2 H) 2.5 (s, 6 H) 2.5 (s, 2 H) 2.9 (m, 5 H) 3.3 (m, 3 H) 7.8 (s, 2 H) 9.1 (s, 1H).

### EXAMPLE 8

### 3-(4-(5-(1-cyclopropyl-7,7-dimethyl-5,6,7,8-tetrahydroisoquinolin-4-yl)-1,2,4-oxadiazol-3-yl)-2,6-dimethylphenyl)propanoic acid

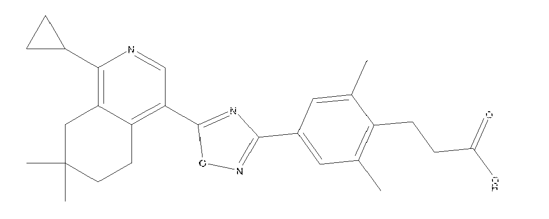

Obtained (90% yield) from Preparation 26 following the general method 4.
LRMS: m/z 446 (M+1)⁺
Retention time: 21.77 min (Method C)
¹H NMR (200 MHz, DMSO-D₆) δ ppm 1.1 (m, 4 H) 1.0 (s, 6 H) 1.6 (t, *J*=6.1 Hz, 2 H) 2.3 (m, 3 H) 2.4 (s, 6 H) 2.7 (s, 2 H) 2.9 (m, 2 H) 3.2 (t, *J*=6.1 Hz, 2 H) 7.7 (s, 2 H) 8.9 (s, 1 H)

### EXAMPLE 9

### 3-(4-(5-(7,7-dimethyl-5,6,7,8-tetrahydroisoquinolin-4-yl)-1,2,4-oxadiazol-3-yl)-2,6-dimethylphenyl)propanoic acid

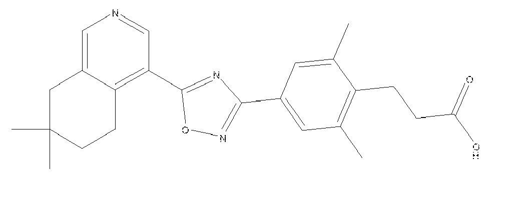

Obtained (88%) from Preparation 29 following the general method 4.
LRMS: m/z 406 (M+1)⁺
Retention time: 19.75 min (Method C)
¹H NMR (200 MHz, DMSO-D₆) δ ppm 1.0 (s, 6 H) 1.7 (t, *J*=6.6 Hz, 2 H) 2.4 (m, 2 H) 2.4 (s, 6 H) 2.7 (s, 2 H) 2.9 (dd, *J*=9.8, 7.0 Hz, 2 H) 3.3 (t, *J*=6.4 Hz, 2 H) 7.7 (s, 2 H) 8.6 (s, 1H) 9.1 (s, 1H)

### EXAMPLE 10

### 2-(4-(5-(1-cyclopropyl-7,7-dimethyl-5,6,7,8-tetrahydroisoquinolin-4-yl)-1,2,4-oxadiazol-3-yl)-2,6-dimethylphenyl)ethanamine

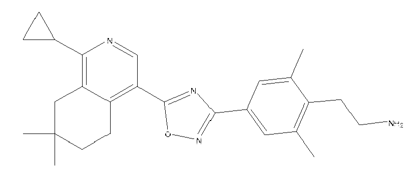

Obtained (20% yield) from Example 8 following the general method 6.
LRMS: m/z 416 (M+1)⁺
Retention time: 15.62 min (Method C)
¹H NMR (200 MHz, CDCl₃) δ ppm 1.1 (m, 4 H) 1.1 (s, 6 H) 1.6 (t, J=6.6 Hz, 2 H) 2.1 (m, 1H) 2.4 (s, 6 H) 2.7 (s, 2 H) 3.1 (m, 3 H) 3.3 (t, *J*=6.4 Hz, 2 H) 3.7 (m, 1H) 4.5 (s, 2 H) 7.8 (s, 2 H) 8.9 (s, 1H)

### EXAMPLE 11

### 2-(4-(5-(7,7-dimethyl-5,6,7,8-tetrahydroisoquinolin-4-yl)-1,2,4-oxadiazol-3-yl)-2,6-dimethylphenyl)ethanamine

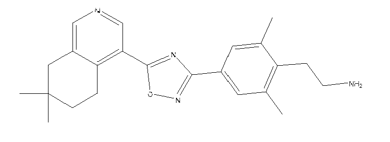

Obtained (43% yield) from Example 9 following the general method 6.
LRMS: m/z 377 (M+1)⁺
Retention time: 12.90 min (Method C)
¹H NMR (200 MHz, CDCl₃) δ ppm 1.0 (s, 6 H) 1.7 (m, 2 H) 2.5 (s, 6 H) 2.6 (s, 2 H) 2.9 (s, 4 H) 3.3 (t, *J*=6.6 Hz, 2 H) 7.8 (s, 2 H) 8.5 (s, 1H) 9.1 (s, 1H)

### EXAMPLE 12

### 3-(4-(5-(7,7-dimethyl-5,6,7,8-tetrahydroisoquinolin-4-yl)-1,2,4-oxadiazol-3-yl)-3-methylphenyl)propanoic acid

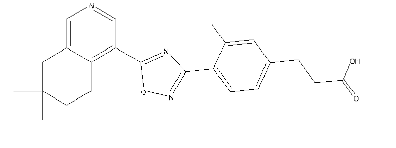

Obtained (65% yield) from Preparation 34 following the general method 3.
LRMS: m/z 392 (M+1)⁺
Retention time: 19.18 min (Method C)
1H NMR (400 MHz, DMSO-D6) δ ppm 1.0 (s, 6 H) 1.6 (t, *J*=6.7 Hz, 2 H) 2.5 (m, 2 H) 2.6 (s, 3 H) 2.6 (s, 2 H) 2.9 (t, *J*=7.4 Hz, 2 H) 3.2 (t, *J*=6.7 Hz, 2 H) 7.3 (d, *J*=8.2 Hz, 1 H) 7.3 (s, 1H) 7.9 (d, J=8.2 Hz, 1H) 8.5 (s, 1H) 9.0 (s, 1H)

### EXAMPLE 13

### 2-(4-(5-(7,7-dimethyl-5,6,7,8-tetrahydroisoquinolin-4-yl)-1,2,4-oxadiazol-3-yl)-3-methylphenyl)ethanamine

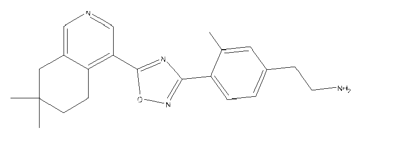

Obtained (22% yield) from Example 12 following the general method 6.
LRMS: m/z 363 (M+1)⁺
Retention time: 12.41 min (Method C)
1H NMR (200 MHz, DMSO-D6) δ ppm 1.0 (s, 6 H) 1.7 (t, *J*=6.1 Hz, 2 H) 2.6 (s, 3 H) 2.7 (s, 2 H) 3.0 (m, 4 H) 3.3 (t, *J*=5.9 Hz, 2 H) 7.3 (m, 2 H) 8.0 (d, *J*=7.4 Hz, 2 H) 8.1 (bs, NH₃⁺) 8.6 (s, 1H) 9.1 (s, 1H)

### EXAMPLE 14

### 2-(3-(4-(5-(7,7-dimethyl-5,6,7,8-tetrahydroisoquinolin-4-yl)-1,2,4-oxadiazol-3-yl)-2,6-dimethylphenyl)propanamido)ethanoic acid

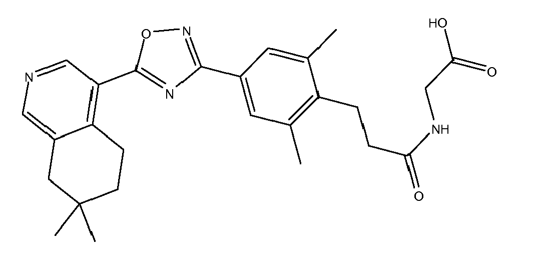

Obtained (78% yield) from Preparation 35 following the general method 4.
LRMS: m/z 463 (M+1)⁺
Retention time: 18.46 min (Method C)
1H NMR (200 MHz, DMSO-D6) δ ppm 1.0 (s, 6 H) 1.7 (m, 2 H) 2.3 (m, 2 H) 2.4 (s, 6 H) 2.7 (s, 2 H) 2.9 (m, 2 H) 3.3 (m, 2 H) 3.8 (d, *J*=5.9 Hz, 2 H) 7.7 (s, 1H) 8.3 (m, 1H) 8.6 (s, 1H) 9.1 (s, 1H)

### EXAMPLE 15

### 3-(4-(5-(7,7-dimethyl-5,6,7,8-tetrahydroisoquinolin-4-yl)-1,2,4-oxadiazol-3-yl)phenyl)propanoic acid

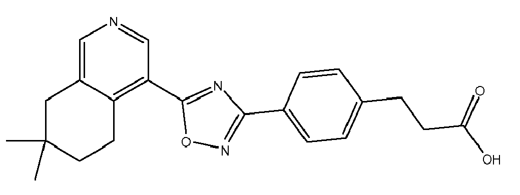

Obtained (80% yield) from Preparation 36 following the general method 3.
LRMS: m/z 378 (M+1)⁺
Retention time: 18.61 min (Method C)
1H NMR (200 MHz, DMSO-D6) δ ppm 1.0 (s, 6 H) 1.6 (t, *J*=6.6 Hz, 2 H) 2.5 (m, *J*=2.0 Hz, 2 H) 2.6 (m, *J*=7.4 Hz, 2 H) 2.9 (m, *J*=7.8 Hz, 2 H) 3.2 (t, *J*=6.4 Hz, 2 H) 7.5 (d, *J*=7.8 Hz, 2 H) 8.0 (d, *J*=8.2 Hz, 2 H) 8.5 (s, 1H) 9.0 (s, 1H)

### EXAMPLE 16

### 4-(4-(5-(7,7-dimethyl-5,6,7,8-tetrahydroisoquinolin-4-yl)-1,2,4-oxadiazol-3-yl)phenyl)butanoic acid

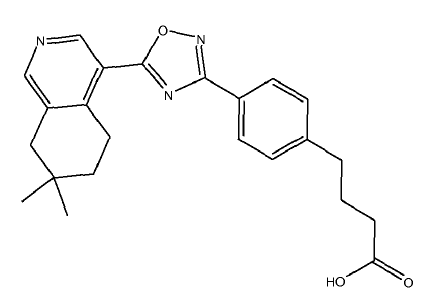

Obtained (41% yield) from Preparation 41 following the general method 3.
LRMS: m/z 392(M+1)⁺
Retention time: 19.31 min (Method C)
1H NMR (200 MHz, DMSO-D6) δ ppm 1.0 (s, 6 H) 1.7 (m, 2 H) 1.8 (m, 2 H) 2.3 (t, *J*=7.4 Hz, 2 H) 2.7 (s, 2 H) 2.7 (m, 2 H) 3.3 (m, 2 H) 7.4 (d, *J*=8.2 Hz, 2 H) 8.0 (d, *J*=8.2 Hz, 2 H) 8.6 (s, 1H) 9.1 (s, 1H)

### EXAMPLE 17

### 2-(4-(5-(7,7-dimethyl-5,6,7,8-tetrahydroisoquinolin-4-yl)-1,2,4-oxadiazol-3-yl)phenyl)ethanamine

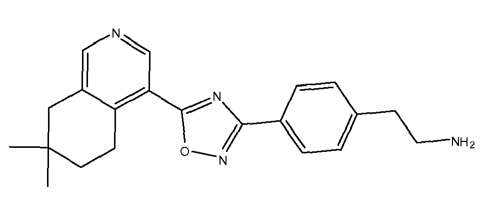

Obtained (47% yield) from Example 15 following the general method 6.
LRMS: m/z 349 (M+1)⁺
Retention time: 11.69 min (Method C)
1H NMR (200 MHz, DMSO-D6) δ ppm 1.0 (m, 6 H) 1.7 (t, *J*=6.4 Hz, 2 H) 2.7 (s, 2 H) 3.0 (m, *J*=12.9 Hz, 4 H) 3.3 (d, *J*=6.4 Hz, 2 H) 7.5 (d, *J*=8.2 Hz, 2 H) 8.1 (d, *J*=8.2 Hz, 2 H) 8.2 (s, 2 H) 8.6 (s, 1H) 9.1 (s, 1H)

### EXAMPLE 18

### 6-(5-(7,7-dimethyl-5,6,7,8-tetrahydroisoquinolin-4-yl)-1,2,4-oxadiazol-3-yl)-1,2,3,4-tetrahydronaphthalen-2-amine

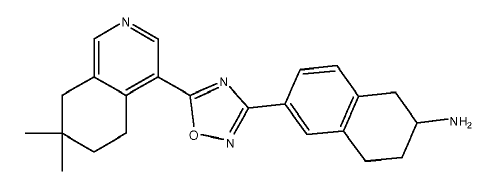

Obtained (62% yield) from Preparation 45 following the general method 4. In this case the fumarate salt was isolated by dissolving the free base with dioxane and adding 1 eq of fumaric acid. The mixture was stirred overnight at r.t. The solid was filtered off and washed with dioxane.
LRMS: m/z 375 (M+1)⁺
Retention time: 12.25 min (Method C)
1H NMR (400 MHz, DMSO-D6) δ ppm 1.0 (m, 6 H) 1.6 (t, *J*=6.5 Hz, 2 H) 1.8 (m, *J*=11.7, 5.5 Hz, 2 H) 2.1 (s, 2 H) 2.6 (s, 2 H) 2.9 (m, 5 H) 3.2 (m, 2 H) 7.4 (d, *J*=8.2 Hz, 1H) 7.9 (m, 2 H) 8.5 (s, 1H) 9.0 (s, 1H)

### EXAMPLE 19

### 3-(4-(5-(7,7-dimethyl-5,6,7,8-tetrahydroisoquinolin-4-yl)-1,2,4-oxadiazol-3-yl)-2,6-dimethylphenyl)propanamide

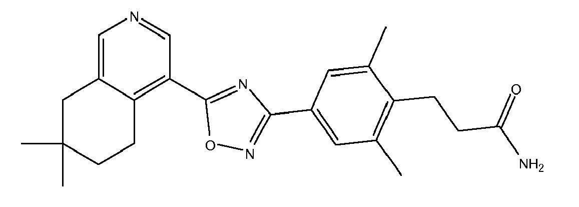

Obtained (44% yield) from Example 9 following the general method 5.
LRMS: m/z 405 (M+1)⁺
Retention time: 18.28 min (Method C)
1H NMR (200 MHz, DMSO-D6) δ ppm 1.0 (s, 6 H) 1.7 (m, 2 H) 2.2 (m, 2 H) 2.4 (s, 6 H) 2.7 (s, 2 H) 2.9 (m, 2 H) 3.3 (m, 2 H) 7.1 (d, *J*=108.1 Hz, 2 H) 7.7 (s, 2 H) 8.7 (s, 1H) 9.1 (s, 1H)

### EXAMPLE 20

### 2-(6-(5-(7,7-dimethyl-5,6,7,8-tetrahydroisoquinolin-4-yl)-1,2,4-oxadiazol-3-yl)-1,2,3,4-tetrahydronaphthalen-2-ylamino)ethanoic acid

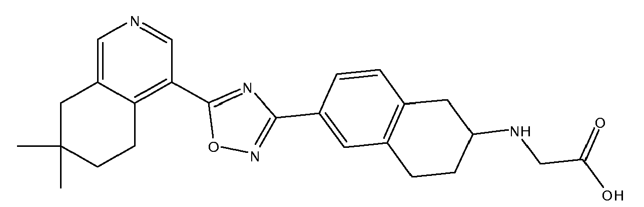

Obtained (42% yield) from Preparation 50 following the general method 3.
LRMS: m/z 433 (M+1)⁺
Retention time: 14.98 min (Method C)
1H NMR (400 MHz, DMSO-D6) δ ppm 1.0 (s, 6 H) 1.6 (t, *J*=6.3 Hz, 2 H) 2.2 (s, 2 H) 2.6 (s, 2 H) 2.9 (m, 3 H) 3.0 (m, *J*=16.4 Hz, 2 H) 3.2 (m, 2 H) 3.3 (s, 2 H) 7.3 (d, *J*=7.4 Hz, 1H) 7.8 (m, 2 H) 8.5 (s, 1H) 9.0 (s, 1H)

### EXAMPLE 21

### 3-(4-(5-(7,7-dimethyl-5,6,7,8-tetrahydroisoquinolin-4-yl)-1,2,4-oxadiazol-3-yl)-2,6-dimethylphenyl)propan-1-amine

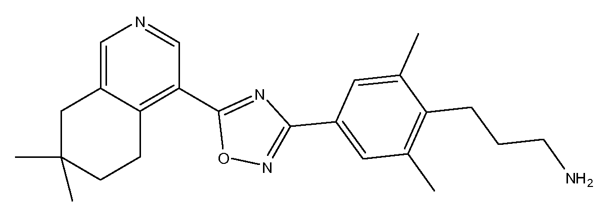

Obtained (21% yield) from Example 19 following the general method 7.
LRMS: m/z 391 (M+1)⁺
Retention time: 13.82 min (Method C)
1H NMR (200 MHz, DMSO-D6) δ ppm 1.0 (m, 6 H) 1.7 (m, 4 H) 2.4 (s, 6 H) 2.7 (s, 2 H) 2.7 (m, 2 H) 2.9 (m, 2 H) 3.3 (m, 2 H) 7.8 (s, 2 H) 8.0 (s, 3 H) 8.6 (s, 1H) 9.1 (s, 1H)

### EXAMPLE 22

### 3-(4-(5-(7,7-dimethyl-5,6,7,8-tetrahydroisoquinolin-4-yl)-1,2,4-oxadiazol-3-yl)-2,6-dimethylphenyl)propane-1,2-diol

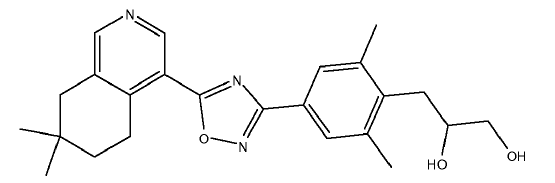

To a solution of Preparation 51 (70mg, 0.19mmol) in a mixture of THF/tert-butanol (1.5mL/0.23mL) was added 4-methylmorpholine 4-oxide (44mg, 0.37mmol) and osmium(VIII) oxide (23 µl, 0.004mmol). The reaction mixture was stirred overnight at r.t. Then 40% solution of Na2SO3 was added and the mixture stirred for 30min. Ethyl acetate was added and the organic layer separated, washed twice with water, dried over magnesium sulphate and concentrated to give the title compound (92% yield).
LRMS: m/z 408 (M+1)⁺
Retention time: 18.30 min (Method C)
1H NMR (200 MHz, CHLOROFORM-D) δ ppm 1.1 (s, 6 H) 1.7 (t, *J*=6.6 Hz, 2 H) 2.4 (m, *J*=8.2 Hz, 6 H) 2.6 (s, 2 H) 2.9 (m, 2 H) 3.3 (t, *J*=6.6 Hz, 2 H) 3.7 (m, 2 H) 4.0 (m, 1 H) 7.8 (s, 2 H) 8.4 (s, 1H) 9.0 (s, 1H)

### EXAMPLE 23

### N-(3-(4-(5-(7,7-dimethyl-5,6,7,8-tetrahydroisoquinolin-4-yl)-1,2,4-oxadiazol-3-yl)-2,6-dimethylphenyl)propyl)methanesulfonamide

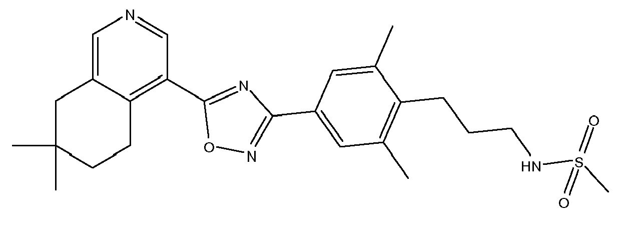

To a solution of Example 21 (75mg, 0.19mmol) in DCM (10mL) was added triethylamine (94 µL 3.51 mmol) and the mixture stirred for 5 min. Then methanesulfonyl chloride (17µL, 0.22mol) was added and the reaction mixture stirred at room temperature for 6h. Ethyl acetate was added and washed twice with water and once with brine. This organic layer was dried over magnesium sulphate and concentrated to give a white solid which was washed with diisopropyl ether and dried in the vacuum oven (66% yield).
LRMS: m/z 469 (M+1)⁺
Retention time: 19.47 min (Method C)
1H NMR (200 MHz, CHLOROFORM-D) δ ppm 1.0 (s, 6 H) 1.8 (m, 4 H) 2.4 (s, 6 H) 2.6 (s, 2 H) 2.8 (m, 2 H) 3.0 (s, 3 H) 3.3 (m, 4 H) 4.4 (t, *J*=5.9 Hz, 1H) 7.8 (s, 2 H) 8.5 (s, 1 H) 9.1 (s, 1H)

### EXAMPLE 24

### N-(4-(5-(7,7-dimethyl-5,6,7,8-tetrahydroisoquinolin-4-yl)-1,2,4-oxadiazol-3-yl)-2,6-dimethylphenethyl)-2,2-difluoroethanamine

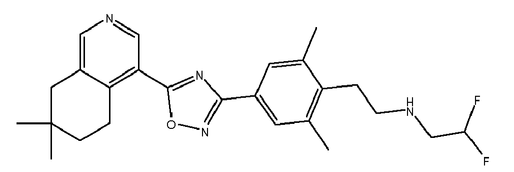

Obtained (50% yield) from Preparation 54 and 2,2-difluoroethanamine following the experimental procedure described for Preparation 50 and purified by the General purification method.
LRMS: m/z 441 (M+1)⁺
Retention time: 13.94 min (Method C)
1H NMR (200 MHz, DMSO-D6) δ ppm 1.0 (s, 6 H) 1.7 (m, 2 H) 2.5 (s, 6 H) 2.7 (s, 2 H) 3.1 (m, 4 H) 3.2 (m, 2 H) 3.6 (m, 2 H) 6.5 (dd, 1H) 7.8 (s, 2 H) 8.6 (s, 1H) 9.1 (s, 1H) 10.1 (s, 2 H)

### EXAMPLE 25

### 1-(4-(5-(7,7-dimethyl-5,6,7,8-tetrahydroisoquinolin-4-yl)-1,2,4-oxadiazol-3-yl)-2,6-dimethylphenethyl)azetidine-3-carboxylic acid

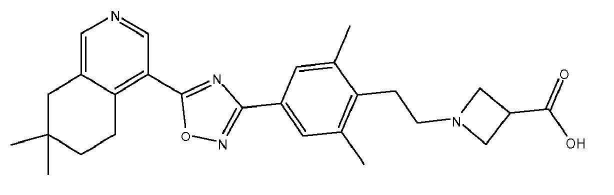

Obtained (60% yield) from Preparation 54 and azetidine-3-carboxylic acid following the experimental procedure described for Preparation 50 and purified by the General purification method.
LRMS: m/z 461 (M+1)⁺
Retention time: 14.95 min (Method C)
1H NMR (200 MHz, DMSO-D6) δ ppm 1.0 (s, 6 H) 1.7 (m, 2 H) 2.5 (s, *J*=9.4 Hz, 6 H) 2.7 (s, 2 H) 3.0 (m, 2 H) 3.3 (m, 4 H) 3.6 (s, *J*=3.9 Hz, 1H) 3.7 (m, 2 H) 4.1 (m, 2 H) 7.8 (s, 2 H) 8.7 (s, 1H) 9.1 (s, 1H)

### EXAMPLE 26

### 3-(5-(5-(7,7-dimethyl-5,6,7,8-tetrahydroisoquinolin-4-yl)-1,2,4-oxadiazol-3-yl)-3-ethyl-6-methylpyridin-2-yl)propanoic acid

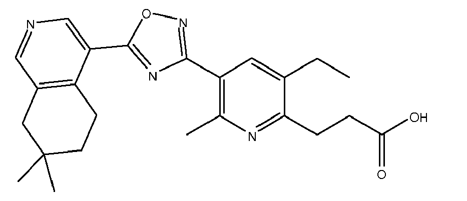

Obtained (82% yield) from Preparation 55 following the general method 3.
LRMS: m/z 421 (M+1)⁺
Retention time: 18.74 min (Method C)
1H NMR (300 MHz, CHLOROFORM-D) δ ppm 1.1 (s, 6 H) 1.3 (t, *J*=7.6 Hz, 3 H) 1.7 (m, *J*=6.6, 6.6 Hz, 2 H) 2.7 (s, 2 H) 2.8 (q, *J*=7.7 Hz, 2 H) 2.9 (m, 2 H) 2.9 (s, 3 H) 3.3 (m, 4 H) 8.3 (s, 1H) 8.5 (s, 1H) 9.2 (s, 1H)

### EXAMPLE 27

### 3-(2-chloro-4-(5-(7,7-dimethyl-5,6,7,8-tetrahydroisoquinolin-4-yl)-1,2,4-oxadiazol-3-yl)phenylsulfonamido)propanoic acid

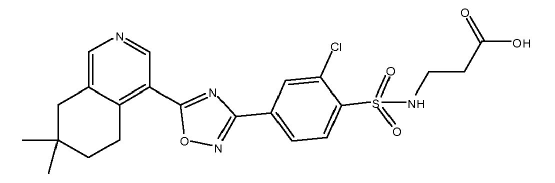

Obtained (84% yield) from Preparation 60 following the general method 4.
LRMS: m/z 491, 493 (M+1)⁺
Retention time: 18.02 min (Method C)
1H NMR (200 MHz, DMSO-D6) δ ppm 1.0 (s, 6 H) 1.7 (t, *J*=6.4 Hz, 2 H) 2.4 (t, *J*=6.8 Hz, 2 H) 2.7 (s, 2 H) 3.1 (m, 2 H) 3.3 (m, *J*=6.6, 6.6 Hz, 2 H) 8.2 (m, 3 H) 8.6 (s, 1H) 9.1 (s, 1H)

### EXAMPLE 28

### 3-(4-(5-(7,7-dimethyl-5,6,7,8-tetrahydroisoquinolin-4-yl)-1,2,4-oxadiazol-3-yl)phenylsulfonamido)propanoic acid

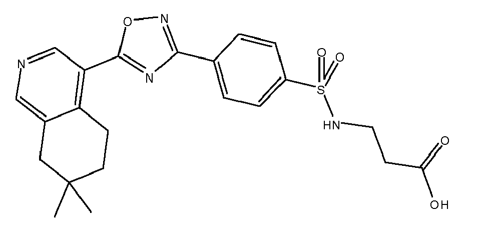

Obtained (44% yield) from Preparation 63 following the general method 4.
LRMS: m/z 457 (M+1)⁺
Retention time: 17.17 min (Method C)
1H NMR (200 MHz, DMSO-D6) δ ppm 1.0 (s, 6 H) 1.7 (t, *J*=6.4 Hz, 2 H) 2.4 (t, *J*=6.8 Hz, 2 H) 2.7 (s, 2 H) 3.0 (m, 2 H) 3.3 (t, *J*=6.4 Hz, 2 H) 7.9 (t, *J*=6.2 Hz, 1H) 8.0 (d, *J=*8.6 Hz, 2 H) 8.3 (d, *J*=8.2 Hz, 2 H) 8.6 (s, 1H) 9.1 (s, 1H)

### EXAMPLE 29

### 3-(4-(5-(1-amino-7,7-dimethyl-5,6,7,8-tetrahydroisoquinolin-4-yl)-1,2,4-oxadiazol-3-yl)-2,6-dimethylphenyl)propane-1,2-diol

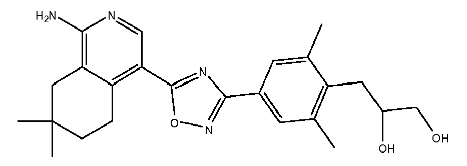

Obtained (90% yield) from Preparation 66 following the experimental procedure described for Example 22.
LRMS: m/z 423 (M+1)⁺
Retention time: 14.26 min (Method C)
1H NMR (300 MHz, DMSO-D6) δ ppm 1.0 (s, 6 H) 1.6 (t, *J*=6.5 Hz, 2 H) 2.2 (s, 2 H) 2.4 (s, 6 H) 2.7 (dd, *J*=13.5, 8.8 Hz, 1H) 2.9 (dd, *J*=13.7, 3.6 Hz, 1H) 3.1 (t, *J*=6.3 Hz, 2 H) 3.4 (m, 4 H) 3.6 (m, 2 H) 7.3 (s, 1H) 7.7 (s, 2 H) 8.6 (m, 1H)

### EXAMPLE 30

### 4-(4-(5-(7,7-dimethyl-5,6,7,8-tetrahydroisoquinolin-4-yl)-1,2,4-oxadiazol-3-yl)-2,6-dimethylphenyl)butanoic acid

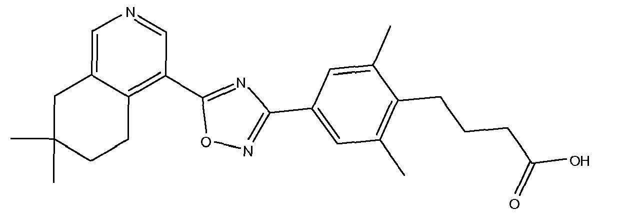

Obtained (35% yield) from Preparation 70 following the general method 3.
LRMS: m/z 420 (M+1)⁺
Retention time: 21,34 min (Method C)
1H NMR (300 MHz, DMSO-D6) δ ppm 1.0 (s, 6 H) 1.7 (t, *J*=6.7 Hz, 4 H) 2.4 (m, 8 H) 2.7 (m, 4 H) 3.3 (t, J=6.5 Hz, 2 H) 7.7 (s, 2 H) 8.6 (s, 1H) 9.1 (s, 1H)

### EXAMPLE 31

### 1-(4-(5-(1-amino-7,7-dimethyl-5,6,7,8-tetrahydroisoquinolin-4-yl)-1,2,4-oxadiazol-3-yl)-2,6-dimethylphenethyl)azetidine-3-carboxylic acid

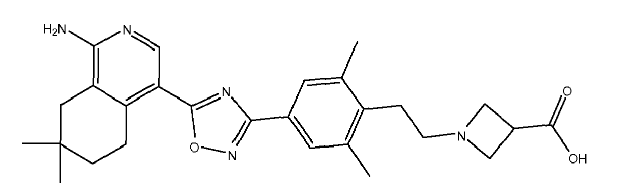

Obtained (13% yield) from Preparation 76 and azetidine-3-carboxylic acid following the experimental procedure described for Preparation 50 and purified by General purification method.
LRMS: m/z 476 (M+1)⁺
Retention time: 11.58 min (Method C) 1H NMR (400 MHz, DMSO-D6) δ ppm 1.0 (s, 6 H) 1.6 (t, *J*=6.5 Hz, 2 H) 2.3 (s, 2 H) 2.4 (s, 6 H) 2.9 (m, 2 H) 3.2 (m, 2 H) 3.7 (m, 1H) 4.2 (m, 6 H) 7.7 (s, 2 H) 8.6 (s, 1H) 11.0 (s, 1H) 11.4 (s, 1H)

### PHARMACOLOGICAL ACTIVITY

### 35S-GTP-g binding assay:

The effect of the compounds was measured using a 35S-GTPyS binding assay. Briefly, membranes were incubated in a buffer containing 20mM HEPES pH 7.4, 100mM NaCl, 10mM MgCl2, 10µM GDP, 50µg/mL saponin and 0.2% fatty acid-free BSA at various concentrations (0.1nM-10µM) and 0.1nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffer. After drying the filter plates scintillant liquid were added to each well and 35S-GTPyS binding was measured on a Trilux Scintillation Counter.

The results are shown in Table 1.

**Table 1**

| **EXAMPLES** | **EC₅₀ (nM)** |
|---|---|
| 6 | 29.0 |
| 7 | 10.8 |
| 8 | 28.5 |
| 22 | 19.0 |
| 24 | 19.4 |
| 25 | 36.7 |

The tetrahidroisoquinolyl derivatives of the invention may also be combined with other active compounds in the treatment of diseases known to be susceptible to improvement by treatment with a sphingosine-1-phosphate receptor agonist (S1P1).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) beta interferons such as Betaseron, Avonex or Rebif, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide, (i) fumaric acid esters, such as *BG-12*, (j) immunomodulators such as Laquinimod, (k) anti-CD20 monoclonal antibodies such as Rituximab, Ocrelizumab Ofatumumab or TRU-015, (l) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as CP-690550 or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162

The combinations of the invention may be used in the treatment of disorders which are susceptible to amelioration by sphingosine-1-phosphate receptors agonists (S1P1). Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the NOVOLIZER ® SD2FL or GENUAIR ® which are described in the following patent applications: WO 97/000703, WO 03/000325 and WO 03/061742. Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of 3-(4-(5-(1-ethyl-7,7-dimethyl-5,6,7,8-tetrahydroisoquinolin-4-yl)-1,2,4-oxadiazol-3-yl)-2,6-dimethylphenyl)propanamide (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 3-(4-(5-(1-ethyl-7,7-dimethyl-5,6,7,8-tetrahydroisoquinolin-4-yl)-1,2,4-oxadiazol-3-yl)-2,6-dimethylphenyl)propanamide (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I), or a pharmaceutically acceptable salt or N-oxide thereof, 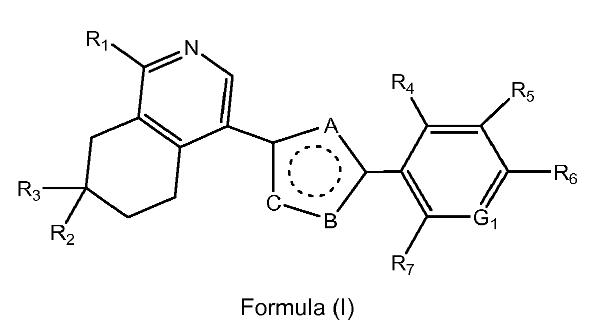 wherein
A is selected from the group consisting of -N-, -O- and -S-;
B and C independently are selected from the group consisting of -N- and -O-, with the proviso that at least two of A, B and C are nitrogen atoms;
G¹ is selected from the group consisting of nitrogen atoms and -CR^{c}- groups, wherein R^{c} represents a hydrogen atom, a halogen atom, a C₁₋₄ alkyl group or a C₁₋₄ alkoxy group;
R¹ is selected from the group consisting of hydrogen atoms, C₁₋₄ alkyl groups, C₁₋₄ alkoxy groups, C₃₋₄ cycloalkyl groups, and -NR^{d}R^{e} groups wherein R^{d} and R^{e} are independently selected from hydrogen atoms and C₁₋₄ alkyl groups;
R² and R³ are independently selected from the group consisting of hydrogen atoms and C₁₋₄ alkyl groups;
R⁴, R⁵ and R⁷ are independently selected from the group consisting of hydrogen atoms, halogen atoms, C₁₋₄ alkyl groups, C₁₋₄ alkoxy groups and C₁₋₄ haloalkyl groups;
R⁶ represents a C₁₋₄ alkyl group or a C₁₋₄ hydroxyalkyl group; or R⁶ is selected from the group consisting of -S(O)₂-NR^{a}R^{b} groups, -(CR^{f}R^{g})ₙ-(CR^{h}Rⁱ)ₓ-(CR^{j}R^{k})_{y}-NR^{a}R^{b} groups, -(CH₂)ₙ-NR^{a}R^{b} groups, -O-(CH₂)ₙ-NR^{a}R^{b} groups, -(CH₂)ₙ-COOH groups, -(CH₂)ₙ-NR^{a}-CO-R^{b'} groups, -(CH₂)ₙ-NR^{a}-(CH₂)ₚ-(NH)_{q}-SO-CH₃ groups and -(CH₂)ₙ-CO-NR^{a}R^{b} groups, wherein
n, p, x and y are each independently integers from 0 to 3,
q is 0 or 1,
R^{f}, R^{g}, R^{h}, Rⁱ, R^{j} and R^{k} independently represent hydrogen atoms or halogen atoms,
R^{b'} is selected from the group consisting of methylsulphonyl groups, C₁₋₄ alkyl groups, C₁₋₄ hydroxyalkyl groups, C₁₋₄ carboxyalkyl groups, and C₁₋₄ haloalkyl groups;
R^{a} and R^{b} are independently selected from the group consisting of hydrogen atoms, methylsulphonyl groups, C₁₋₄ alkyl groups, C₁₋₄ hydroxyalkyl groups, C₁₋₄ carboxyalkyl groups, and C₁₋₄ haloalkyl groups, or
R^{a} and R^{b} or R^{a} and R^{b}' together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by -NHR' wherein R' represents a hydrogen atom or a C₁₋₄ carboxyalkyl group.

2. A compound according to claim 1, wherein
R⁶ represents a C₁₋₄ alkyl group, or a C₁₋₄ hydroxyalkyl group, or
R⁶ is selected from the group consisting of -S(O)₂-NR^{a}R^{b} groups, -(CH₂)ₙ-NR^{a}R^{b} groups, -(CH₂)ₙ-COOH groups, and -(CH₂)ₙ-CO-NR^{a}R^{b} groups, wherein,
n is an integer from 0 to 3,
R^{a} and R^{b} are independently selected from the group consisting of hydrogen atoms, methylsulphonyl groups and C₁₋₄ carboxyalkyl groups, C₁₋₄ alkyl groups and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by -NHR' wherein R' represents a hydrogen atom or a C₁₋₄ carboxyalkyl group.

3. A compound according to claim 1 or claim 2, wherein A is selected from the group consisting of -N- and -O-.

4. A compound according to any one of claims 1 to 3, wherein A represents -N-.

5. A compound according to any one of the preceding claims, wherein G¹ represents a -CR^{c}- group, wherein R^{c} represents a hydrogen atom, chlorine atom, fluorine atom or a C₁₋₄ alkyl group.

6. A compound according to any one of claims 1 to 5, wherein R¹ represents a hydrogen atom, a C₁₋₄ alkyl group or a C₃₋₄ cycloalkyl group.

7. A compound according to any one of the preceding claims, wherein R² represents a C₁₋₄ alkyl group.

8. A compound according to any one of the preceding claims, wherein R³ represents a C₁₋₄ alkyl group.

9. A compound according to any one of claims 1 to 8, wherein R⁴ represents a hydrogen atom.

10. A compound according to any one of the preceding claims, wherein R⁷ represents a hydrogen atom.

11. A compound according to any one of the preceding claims, wherein R⁵ represents a hydrogen atom or a C₁₋₄ alkyl group.

12. A compound according to any one of claims 1 or 3 to 11, wherein R⁶ represents a C₁₋₄ alkyl group, or a C₁₋₄ hydroxyalkyl group; or R⁶ is selected from the group consisting of -(CH₂)ₙ-NR^{a}R^{b} groups, -(CH₂)ₙ-COOH groups, -(CH₂)ₙ-NR^{a}-(CH₂)ₚ-(NH)_{q}-SO-CH₃ groups and -(CH₂)ₙ-CO-NR^{a}R^{b} groups, wherein
n and p are each independently integers from 0 to 3,
q is 0 or 1,
R^{a} and R^{b} are independently selected from the group consisting of hydrogen atoms, methylsulphonyl groups, C₁₋₄ carboxyalkyl groups, C₁₋₄ alkyl groups and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group.

13. A compound according to claim 1, wherein:
A represents -N-;
G¹ represents a -CR^{c}- group, wherein R^{c} represents a hydrogen atom or C₁₋₄ alkyl group;
R² and R³ independently represent C₁₋₄ alkyl groups;
R⁴, R⁵ and R⁷ are independently selected from the group consisting of hydrogen atoms and C₁₋₄ alkyl groups;
R⁶ represents a C₁₋₄ alkyl group or a C₁₋₄ hydroxyalkyl group, or R⁶ is selected from the group consisting of -S(O)₂-NR^{a}R^{b} groups, -(CH₂)ₙ-NR^{a}R^{b} groups, -(CH₂)ₙ-COOH groups, -(CH₂)ₙ-NR^{a}-(CH₂)ₚ-(NH)_{q}-SO-CH₃ groups and -(CH₂)ₙ-CO-NR^{a}R^{b} groups, wherein
n and p are each independently integers from 0 to 3,
q is 0 or 1,
R^{a} and R^{b} independently are selected from the group consisting of hydrogen atoms, methylsulphonyl groups, C₁₋₄ carboxyalkyl groups, C₁₋₄ alkyl groups and C₁₋₄ haloalkyl groups, or
R^{a} and R^{b} together with the nitrogen atom to which they are attached form a 4 to 6 membered, saturated heterocyclic group, which contains, as heteroatoms, one or two nitrogen atoms and which is substituted by a carboxyl group or a C₁₋₄ carboxyalkyl group; or
R^{c} together with R⁶ form a C₅₋₈ carbocyclic ring optionally substituted by -NHR' wherein R' represents a hydrogen atom or a C₁₋₄ carboxyalkyl group.

14. A compound according to claim one, which is a compound of formula (I'), or a pharmaceutically acceptable salt or N-oxide thereof, 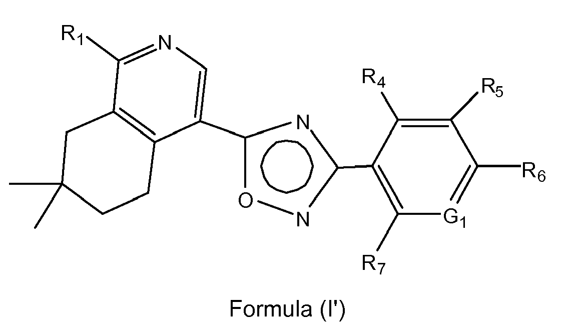 wherein
G¹ is selected from the group consisting of nitrogen atoms and -CR^{c}- groups, wherein R^{c} represents a hydrogen atom, a chlorine atom, or a C₁₋₂ alkyl group;
R¹ is selected from the group consisting of hydrogen atoms, C₁₋₂ alkyl groups, cyclopropyl groups, and -NH₂ groups;
R⁴, R⁵ and R⁷ are independently selected from the group consisting of hydrogen atoms, chlorine atoms, C₁₋₂ alkyl groups;
R⁶ represents a C₁₋₂ alkyl group or a C₁₋₄ hydroxyalkyl group; or R⁶ is selected from the group consisting of -S(O)₂-NHR^{b} groups, -(CH₂)ₙ-NHR^{b} groups, -(CH₂)ₙ-COOH groups, and -(CH₂)ₙ-CO-NHR^{b} groups, wherein
n is 2 or 3,
R^{b} is selected from the group consisting of hydrogen atoms, methylsulphonyl groups, C₁₋₂ carboxyalkyl groups, and C₁₋₂ haloalkyl groups, or
R^{a} and R^{b} together with the nitrogen atom to which they are attached form an azetidine or piperazine group which groups are substituted by a carboxyl group or a C₁₋₂ carboxyalkyl group; or
R^{c} together with R⁶ form a C6 carbocyclic ring substituted by -NHR' wherein R' represents a hydrogen atom or a C₁₋₂ carboxyalkyl group.

15. A compound according to claim 1 which is one of:
(4-(5-(1-ethyl-7,7-dimethyl-5,6,7,8-tetrahydroisoquinolin-4-yl)-1,2,4-oxadiazol-3-yl)phenyl)methanol
4-(5-(1-ethyl-7,7-dimethyl-5,6,7,8-tetrahydroisoquinolin-4-yl)-1,2,4-oxadiazol-3-yl)benzenesulfonamide
3-(4-(4-(5-(1-ethyl-7,7-dimethyl-5,6,7,8-tetrahydroisoquinolin-4-yl)-1,2,4-oxadiazol-3-yl)phenyl)piperazin-1-yl)propanoic acid
3-(4-(5-(1-ethyl-7,7-dimethyl-5,6,7,8-tetrahydroisoquinolin-4-yl)-1,2,4-oxadiazol-3-yl)phenylsulfonamido)propanoic acid
3-(4-(5-(1-ethyl-7,7-dimethyl-5,6,7,8-tetrahydroisoquinolin-4-yl)-1,2,4-oxadiazol-3-yl)-2,6-dimethylphenyl)propanoic acid
3-(4-(5-(1-ethyl-7,7-dimethyl-5,6,7,8-tetrahydroisoquinolin-4-yl)-1,2,4-oxadiazol-3-yl)-2,6-dimethylphenyl)propanamide
2-(4-(5-(1-ethyl-7,7-dimethyl-5,6,7,8-tetrahydroisoquinolin-4-yl)-1,2,4-oxadiazol-3-yl)-2,6-dimethylphenyl)ethanamine
3-(4-(5-(1-cyclopropyl-7,7-dimethyl-5,6,7,8-tetrahydroisoquinolin-4-yl)-1,2,4-oxadiazol-3-yl)-2,6-dimethylphenyl)propanoic acid
3-(4-(5-(7,7-dimethyl-5,6,7,8-tetrahydroisoquinolin-4-yl)-1,2,4-oxadiazol-3-yl)-2,6-dimethylphenyl)propanoic acid
2-(4-(5-(1-cyclopropyl-7,7-dimethyl-5,6,7,8-tetrahydroisoquinolin-4-yl)-1,2,4-oxadiazol-3-yl)-2,6-dimethylphenyl)ethanamine
2-(4-(5-(7,7-dimethyl-5,6,7,8-tetrahydroisoquinolin-4-yl)-1,2,4-oxadiazol-3-yl)-2,6-dimethylphenyl)ethanamine
3-(4-(5-(7,7-dimethyl-5,6,7,8-tetrahydroisoquinolin-4-yl)-1,2,4-oxadiazol-3-yl)-3-methylphenyl)propanoic acid
2-(4-(5-(7,7-dimethyl-5,6,7,8-tetrahydroisoquinolin-4-yl)-1,2,4-oxadiazol-3-yl)-3-methylphenyl)ethanamine
2-(3-(4-(5-(7,7-dimethyl-5,6,7,8-tetrahydroisoquinolin-4-yl)-1,2,4-oxadiazol-3-yl)-2,6-dimethylphenyl)propanamido)ethanoic acid
3-(4-(5-(7,7-dimethyl-5,6,7,8-tetrahydroisoquinolin-4-yl)-1,2,4-oxadiazol-3-yl)phenyl)propanoic acid
4-(4-(5-(7,7-dimethyl-5,6,7,8-tetrahydroisoquinolin-4-yl)-1,2,4-oxadiazol-3-yl)phenyl)butanoic acid
2-(4-(5-(7,7-dimethyl-5,6,7,8-tetrahydroisoquinolin-4-yl)-1,2,4-oxadiazol-3-yl)phenyl)ethanamine
6-(5-(7,7-dimethyl-5,6,7,8-tetrahydroisoquinolin-4-yl)-1,2,4-oxadiazol-3-yl)-1,2,3,4-tetrahydronaphthalen-2-amine
3-(4-(5-(7,7-dimethyl-5,6,7,8-tetrahydroisoquinolin-4-yl)-1,2,4-oxadiazol-3-yl)-2,6-dimethylphenyl)propanamide
2-(6-(5-(7,7-dimethyl-5,6,7,8-tetrahydroisoquinolin-4-yl)-1,2,4-oxadiazol-3-yl)-1,2,3,4-tetrahydronaphthalen-2-ylamino)ethanoic acid
3-(4-(5-(7,7-dimethyl-5,6,7,8-tetrahydroisoquinolin-4-yl)-1,2,4-oxadiazol-3-yl)-2,6-dimethylphenyl)propan-1-amine
3-(4-(5-(7,7-dimethyl-5,6,7,8-tetrahydroisoquinolin-4-yl)-1,2,4-oxadiazol-3-yl)-2,6-dimethylphenyl)propane-1,2-diol
N-(3-(4-(5-(7,7-dimethyl-5,6,7,8-tetrahydroisoquinolin-4-yl)-1,2,4-oxadiazol-3-yl)-2,6-dimethylphenyl)propyl)methanesulfonamide
N-(4-(5-(7,7-dimethyl-5,6,7,8-tetrahydroisoquinolin-4-yl)-1,2,4-oxadiazol-3-yl)-2,6-dimethylphenethyl)-2,2-difluoroethanamine
1-(4-(5-(7,7-dimethyl-5,6,7,8-tetrahydroisoquinolin-4-yl)-1,2,4-oxadiazol-3-yl)-2,6-dimethylphenethyl)azetidine-3-carboxylic acid
3-(5-(5-(7,7-dimethyl-5,6,7,8-tetrahydroisoquinolin-4-yl)-1,2,4-oxadiazol-3-yl)-3-ethyl-6-methylpyridin-2-yl)propanoic acid
3-(2-chloro-4-(5-(7,7-dimethyl-5,6,7,8-tetrahydroisoquinolin-4-yl)-1,2,4-oxadiazol-3-yl)phenylsulfonamido)propanoic acid
3-(4-(5-(7,7-dimethyl-5,6,7,8-tetrahydroisoquinolin-4-yl)-1,2,4-oxadiazol-3-yl)phenylsulfonamido)propanoic acid
3-(4-(5-(1-amino-7,7-dimethyl-5,6,7,8-tetrahydroisoquinolin-4-yl)-1,2,4-oxadiazol-3-yl)-2,6-dimethylphenyl)propane-1,2-diol
4-(4-(5-(7,7-dimethyl-5,6,7,8-tetrahydroisoquinolin-4-yl)-1,2,4-oxadiazol-3-yl)-2,6-dimethylphenyl)butanoic acid
1-(4-(5-(1-amino-7,7-dimethyl-5,6,7,8-tetrahydroisoquinolin-4-yl)-1,2,4-oxadiazol-3-yl)-2,6-dimethylphenethyl)azetidine-3-carboxylic acid
and the pharmaceutically acceptable salts and N-oxides thereof.

16. A compound according to any one of claims 1 to 15 for use in the treatment of a pathological condition or disease susceptible to amelioration by sphingosine-1-phosphate receptors (S1P1) agonists.

17. A compound according to claim 16, wherein the pathological condition or disease is selected from autoimmune diseases, chronic immune and inflammatory diseases, transplant rejection, malignant neoplastic diseases, angiogenic-related disorders, pain, neurological diseases, viral and infectious diseases.

18. A compound according to claim 16 or 17 wherein the pathological condition or disease is selected from multiple sclerosis, transplant rejection, systemic lupus erythematosus, asthma, psoriasis, rheumatoid arthritis, psoriatic arthritis and Crohn's disease.

19. A pharmaceutical composition comprising a compound as defined in any one of claims 1 to 15 in association with a pharmaceutically acceptable diluent or carrier.

20. Use of a compound as defined in any one of claims 1 to 15 for the manufacture of a medicament for the treatment of a pathological condition or disease as defined in any one of claims 16 to 18.

21. A method for treating a subject afflicted with a pathological condition or disease as defined in claims 16 to 18, which comprises administering to said subject a therapeutically effective amount of a compound as defined in any one of claims 1 to 15.

22. A combination product comprising (i) a compound according to any one of claims 1 to 15; and (ii) another compound selected from:
a) Beta interferons such as Betaseron, Avonex or Rebif
b) Immunomodulators such as glatiramer acetate
c) Inhibitors of DNA synthesis and repair, such as Mitoxantrone
d) Anti-alpha 4 integrin antibodies, such as Natalizumab (Tysabri)
e) Alpha 4 integrin antagonists such as R-1295 , TBC-4746, CDP-323, ELND-002, Firategrast and TMC-2003
f) Dyhydrofolate reductase inhibitors, such as Methotrexate or CH-1504
g) Glucocorticoids such as prednisone or methylprednisolone
h) DHODH inhibitors such as teriflunomide
*i)* Fumaric acid esters, such as *BG-12*
*j)* Immunomodulators such as Laquinimod
k) Anti-CD20 monoclonal antibodies such as Rituximab, Ocrelizumab Ofatumumab or TRU-015
l) Anti-CD52 such as alemtuzumab
m) Anti-CD25 such as daclizumab
n) Anti-CD88, such as eculizumab or pexilizumab
o) Calcineurin inhibitors such as cyclosporine A or tacrolimus
p) IMPDH inhibitors, such as mycophenolate mophetyl
q) Cannabinoid receptor agonists such as Sativex
r) Chemokine CCR1 antagonists such as MLN-3897 or PS-031291
s) Chemokine CCR2 antagonists such as INCB-8696
t) Interferon alpha such as Sumiferon MP
u) NF-kappaB activation inhibitors such as FAE and MLN-0415
v) JAK inhibitors such as CP-690550 or INCB018424
w) Syk inhibitors, such as R-112
x) PKC inhibitors, such as NVP-AEB071
y) Phosphosdiesterase IV inhibitors such as GRC-4039
z) P38 Inhibitors such as ARRY-797
aa) MEK inhibitors, such as ARRY-142886 or ARRY-438162
